# EUROPEAN PATENT SPECIFICATION

(11) **EP 4 244 574 B1**
(45) Date of publication and mention of the grant of the patent: **20.08.2025**
(21) Application number: 21806422.8
(22) Date of filing: 11.11.2021
(51) Int. Cl.: G01B 11/06, C03B 9/40, G01B 21/08, G01N 25/72, G01B 21/20

(54) **METHOD FOR INSPECTING HOLLOW GLASS PRODUCTS OF GLASS PRODUCT MATERIAL**
VERFAHREN ZUR INSPEKTION VON HOHLGLASPRODUKTEN AUS GLASPRODUKTMATERIAL
PROCÉDÉ D'INSPECTION DE PRODUITS EN VERRE CREUX EN MATÉRIAU DE PRODUIT EN VERRE

(30) Priority: 11.11.2020 NL 2026865; 14.05.2021 NL 2028215; 14.05.2021 NL 2028216
(43) Date of publication of application: 20.09.2023
(73) Proprietor: Centrum voor Technische Informatica B.V., 9728 JT Groningen (NL)
(72) Inventor: DALSTRA, Joop, 9728 JT Groningen (NL)
(74) Representative: V.O.
(86) International application number: PCT/NL2021/050693
(87) International publication number: WO 2022/103262

(56) References cited:
- EP-A1- 2 743 689
- EP-A1- 3 239 697
- EP-A2- 1 020 703
- EP-A2- 2 333 502
- WO-A1-2019/133504
- DE-B3- 102019 005 487

## Description

The invention relates to a method for inspecting hollow glass products of glass product material, wherein the glass products are manufactured by:
a. heating the glass product material;
b. forming the heated glass product material into at least one glass product in a production flow;
c. cooling the formed glass product.

The invention further relates to a method for producing and inspecting hollow glass products of glass product material, wherein the glass products are at least manufactured by:
a. heating the glass product material;
b. forming the heated glass product material into a glass product in a production flow;
c. cooling the formed glass product.

Also, the invention relates to a system for producing and inspecting glass products of glass product material according to the above-mentioned method for inspecting hollow glass products, wherein the system comprises:
a heating apparatus for carrying out step a.;
a product forming apparatus such as a mould for carrying out step b.;
a cooling apparatus for carrying out step c.; and
a transporting apparatus for transporting glass products formed with the product forming apparatus from the product forming apparatus to the cooling apparatus;
at least one sensor for scanning the glass products formed in step b.; and
a signal processing unit connected with the at least one sensor for processing signals coming from the at least one sensor.

In addition, the invention relates to a system for inspecting glass products of glass product material according to the above-mentioned method for inspecting the glass products, wherein the system comprises:
at least one sensor for scanning the glass products; and
a signal processing unit connected with the at least one sensor for processing signals coming from the at least one sensor.

Such methods and systems are known per se, for instance from WO-2019133504A1. In the known method, using a plurality of sensors, images of the still-hot just-manufactured glass products are made. Due to the sensors being set up around the glass product, with the images a full revolution of the product is covered. The making of such a group of images is carried out repeatedly at different points of time. On each of the images, the intensity of the infrared radiation is visible. By comparing two images made at different points of time of a same part of the product, a decrease of the intensity can be established. If the intensity decreases relatively slowly, it is established that the glass material at that spot is relatively thick. If the intensity decreases relatively fast, it is established that the glass material at that spot is relatively thin. In this manner, a lateral glass thickness distribution of the glass product can be determined. A disadvantage is that this method is relatively inaccurate. For instance, the intensity is also a function of the temperature, which needs to be estimated to be able to determine from the intensity a glass thickness distribution. Furthermore, setting up the sensors around the product is costly and laborious.

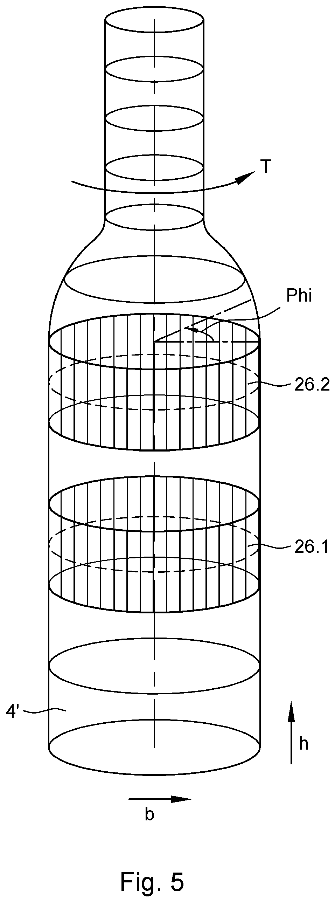

The just-mentioned lateral glass thickness distribution, also referred to by the abbreviation LGD (Lateral Glass Distribution), at a defined height is the set of the wall thicknesses around the circumference of the product (Figure 5). This LGD can be obtained, for instance, by measuring glass wall thicknesses at a defined height h and performing the thickness measurements around the entire circumference of the product. All wall thicknesses of the whole product are the Total Lateral Glass Distribution of the product. A single wall thickness of the product at a defined height h and angle phi (polar coordinates) is an element of the set of wall thicknesses: Lateral Glass Distribution LGD(h,phi). The number of elements in the LGD depends on the, freely to be chosen, measuring resolution of the height and the angle.

The LGD is a very important parameter for the quality of the glass product. The strength of the product is chiefly determined by the thinnest part of a glass wall. In order to prevent breakage in the normal use of the product, the LGD must comply with the specifications of a producer. However, with the current glass production technology, the variation of the Lateral Glass Distribution can range from 35% to 55% of the average glass wall thickness. To arrange for the product to be yet sufficiently strong (minimal reject), this glass thickness variation is compensated for by making the glass wall of extra thick design. As a result, not only does the product become heavier (more glass), but also more base materials are used, it takes more energy to produce the product (melting and annealing) and the transport costs of the glass product become higher due to the extra weight. By minimizing the variation of the lateral glass thickness distribution of the product, the design of the product can be adapted to have a thinner (more constant) glass wall thickness. The product becomes lighter, the production costs fall proportionally, and so do the transport costs (as well as the CO₂, NOₓ emissions depending thereon).

To minimize the variation in the Lateral Glass Distribution in the industrial glass forming process, sensors that are able to determine the LGD in the glass forming process are requisite. Using these sensors, in the production process the root causes of the variation of the LGD can be investigated, for instance to ascertain which process settings or parts of the process are responsible for an unduly large variation in the LGD. If these causes of the variation of the LGD are known, the responsible process steps can be improved, for instance by optimizing the setting by using the measuring data of the sensor. This may also be done automatically with a feedback system (FeedBack loop) to automate the optimum settings so as to obtain a minimal variation of the LGD. Also, improvements may be incorporated in the responsible process steps, to minimize the variation of the LGD.

Object of the invention is to improve the known inspection process and possibly, based on the improved inspection process, to improve the production process.

The method for inspecting hollow glass products according to the invention is characterised in that inspecting the glass products comprises the following steps:
d. transporting the glass products formed in step b. successively along a predetermined path along at least one glass thickness sensor such as a Chromatic Confocal sensor, wherein, using the at least one sensor, of a plurality of the glass products that are transported successively along the at least one sensor, per glass product at least one glass thickness is measured, wherein step d. is carried out between steps b. and c. and wherein in step d. of each glass product of the plurality of glass products the rotational position of the glass product around an axial axis of the glass product relative to the at least one sensor is determined, wherein in a step e. the determined glass thicknesses and the associated rotational positions of the plurality of products are processed in combination for obtaining information about a lateral glass thickness distribution around an axial axis of a virtual glass product that represents the plurality of glass products.

The invention is based on the first insight that a production fault or production deviation in a glass product will typically occur to a comparable extent and at comparable positions in all successively manufactured glass products. When the glass products have been formed in step b., they all have a same rotational position. A deviation in the forming process of the products is then present at this same position in the successively made glass products. When the glass products are transported along the path, however, the products have mutually obtained a more or less random rotational position. This is a consequence of the manner in which glass products are transported after step b. is carried out. Accordingly, when on a glass product a measurement is performed with the at least one sensor, the glass product has a more or less random rotational position relative to the at least one sensor. This means that mutually different products will have mutually different rotational positions relative to each other and hence relative to the at least one sensor when in succession on the products a measurement is performed with the at least one sensor. Each measurement on the glass product from a more or less random viewing angle relative to the glass product thus relates to a measurement on a particular position of the product. By performing measurements on a plurality of products and analysing these measurements in combination with information about the rotational position of the respective products relative to the at least one sensor, an impression can be obtained of what the glass thickness is at different positions of the average manufactured product on which the measurements have been performed. Because generally with different products measuring is done at different positions, with measurements on a sufficient number of products an impression can be obtained of what the glass thickness is at different positions on the glass thickness-wise average manufactured product. This average product is here also called a virtual product. A virtual product is thus a product having a lateral glass thickness distribution that has been composed from measured glass thicknesses obtained by measuring on different products. Each glass thickness at a defined position of the virtual product then corresponds to at least one glass thickness which at a same position has in actual fact been measured on at least one real product (or to an average of glass thicknesses that have been measured on different products at a same position).

Given sufficient measurements on different products having a more or less mutually different rotational position relative to the at least one sensor, thus a lateral glass thickness distribution can be obtained that extends completely around an axial axis of the virtual glass product. And this while only a single sensor needs to be utilized. The result is as if with a plurality of sensors set up in tangential direction of the product around the product, thicknesses are measured at mutually different positions around the axial axis. The difference is, however, that it is not about a plurality of thickness measurements on a single (same) product and spread around that product, but about a plurality of products where per product of the plurality of products at least one thickness measurement on a position of that product is performed. In this way, an impression can be obtained of a lateral glass thickness distribution of an average glass product that is formed.

The lateral glass thickness distribution may be expressed in numbers (for instance a glass thickness at a particular position of the glass product (absolute glass thickness distribution) or a deviation from an average glass thickness (relative glass thickness)).

On the basis of the lateral glass thickness distribution it may be determined for instance whether a glass thickness distribution is within predetermined limits. If this is not the case, for instance the glass products on which measuring has been done with the sensor may be rejected, but it is also possible to adjust a parameter of the glass production process, such as for instance the temperature with which the glass product material is heated in step a. or the forming of the heated glass product material into the glass product in step b. In this forming, for instance moulds may be utilized. Adjusting (also: adapting) step b. may then for instance consist in replacing a mould by a new mould. Also, in step b. troughs may be utilized through which the glass product material flows in the form of a glass gob towards a mould. Such troughs may, for instance in case of an established deviation of a glass thickness distribution, be lubricated with a lubricant. Other adjustments are also possible, of course. These adjustments may then be carried out automatically. It is also possible, however, that some adjustments of step b. are carried out manually.

It holds preferably that the glass thickness distribution comprises absolute values of the glass thickness distribution.

Also, the glass thickness distribution may be shown on a screen by means of a 3D image of a transparent view of the virtual glass product. With each of the above-mentioned possibilities, use can be made of the circumstance that the shape and size of the products on which measuring is done are known beforehand. This is because products are manufactured which have been specified beforehand. To put it differently, the specified dimensions (including shape, size and including wall thicknesses) of the product can be adopted for the virtual product and be corrected on the basis of the measurements on real products, there where the measurements deviate from the specifications.

In this virtual product, the wall thicknesses may then be drawn in or be noted in a table, on the basis of the wall thicknesses and associated rotational positions which have been measured on different products.

In particular it holds that each product is transported in a horizontally directed plane, with the axial axis of the product being vertically directed.

Further, it holds preferably that on a sufficiently large number of products from a production flow, measurements with the at least one sensor are performed in order that the measuring positions of these measurements in the virtual product have neighbouring distances in tangential direction that are each smaller than a predetermined value.

Also, it holds in particular that in step e. the lateral glass thickness distribution is determined in an area of the virtual glass product that extends around the axial axis of the virtual glass product. Here, it may furthermore hold in particular that the step e. is carried out repeatedly for obtaining a lateral glass thickness distribution in a second area of the virtual glass product that extends around the axial axis of the virtual glass product, wherein the first and second area are staggered relative to each other in the axial direction. Here, it holds in particular that the at least one sensor is displaced in the axial direction for obtaining recordings in the different areas.

According to an advanced embodiment of the method, it holds that in step d. with the at least one sensor successively a plurality of wall thicknesses are determined of a glass product that is transported along the at least one sensor so that the determined glass thicknesses relate to different positions of a wall of the glass product, these positions being separate from each other in a direction in which the product is transported in step d. In practice, for instance 300 measurements per second are performed. The transport velocity of a glass product along the at least one sensor is for instance 0.5 m/sec so that measuring points in a glass product are spaced apart approximately 0.5/300 = 0.166 cm measured along the outer surface (assuming that a curvature of the outer surface is not too high, otherwise this distance will be slightly larger). If the product is of substantially cylinder-shaped configuration with a vertical axial axis that is perpendicular to the transport direction and a diameter of 15 cm, with the sensor an area can be covered that at a maximum in the transport direction has a dimension of for instance 5 cm measured along the outer surface of the product. This is because for performing a measurement, a line along a measuring direction of the sensor at a position where the line first intersects the product, an angle with a normal to the surface at said position must be smaller than for instance 5 degrees (at a first possible measurement on a glass product, the angle is then 5 degrees relative to the normal, at a last possible measurement this angle is -5 degrees relative to the normal and halfway the number of measurements the angle is approximately 0 degrees). This is because the sensor is typically an active sensor which emits light and receives reflections for determining the glass thickness. If the angle mentioned is greater than for instance 5 degrees, a reflection of the light emitted by the sensor on the surface of the product will not reach the sensor so that no thickness measurement can be performed. The dimension of 5 cm means that on the product approximately 2*5/0.166 = 60 thickness measurements can be performed (a first measurement at 5 degrees relative to the normal and a last measurement at -5 degrees relative to the normal). For that matter, the measured thicknesses at those points where the angle mentioned is unequal to 0 degrees can be automatically corrected to obtain the in-situ thickness in a radial direction of the glass product. This correction can be computed on the basis of a known geometry of the glass product and the distance over which the product in the step d. has been transported between the measurement at 0 degrees and the measurement at a different angle, as well as a distance between the sensor and the glass product at the moments of measuring with the sensor. The correction at the maximum angle of +/- 5 degrees, however, is relatively small. Further, it holds in that example that the area which in the transport direction has approximately a length of 5 cm only covers a 5/ Pi*15 part of the full circumference of the product. So this is approximately a 0.106 part. To be able to cover the full circumference of the virtual product, therefore, at least 1/0.106 = 9 products are needed on which a measurement is performed. If the products that successively pass the sensor have for instance a random rotational position around their axial axes relative to the sensor, statistically, measuring will have to be done on many more than nine successive products in order for the measurements in combination to cover the virtual product fully around its axial axis. Think for instance of 100 successive products.

In a preferred embodiment of the sensor such as the confocal sensor, it holds that using the at least one sensor a first distance is measured between the at least one sensor and the outer surface of the part of the wall and a second distance is measured between an inner surface of the part of the wall, wherein from the first distance and the second distance the glass thickness of the part of the wall is determined.

In particular, it holds that in step d. the product is transported in a horizontal plane, wherein the at least one sensor is displaced in vertical direction for measuring on the glass product at different heights. Preferably, it holds here that a glass thickness of the product is determined at different heights. Furthermore, it is possible that from the measuring results obtained with the at least one sensor a skew position of the product is determined. This is possible in that in particular with the sensor also a distance from an inner or outer surface of the product to the sensor is determined. By doing this at different heights, a skew position follows. The glass thickness may be determined by, at a point of a wall of the sensor whose normal includes an angle with a measuring direction of the sensor that is smaller than a predetermined value such as for instance 15 degrees, more particularly 5 degrees, determining the difference at that point in distance between the outer wall and the sensor and the difference at the point in distance between the inner wall and the sensor.

According to an advanced embodiment, in step d. use is made of at least two mutually opposite and mutually facing sensors, with the products being transported between the at least two sensors. In this manner, on a product a double measurement can be performed and hence information about glass thickness of the product can be determined that relates to a surface twice as large compared with the method in which only one sensor is used. In particular, it holds here that with a first sensor of the at least two sensors a first glass thickness of a first part of the wall of the glass product is determined and that with a second sensor of the at least two sensors a second glass thickness of a second part of the wall of the glass product is determined, wherein the first part and the second part are on sides of the glass product located opposite to each other.

Furthermore, it holds here in particular that with the first sensor a first distance is measured between the first sensor and an outer surface of a first part of the wall that faces the first sensor and that with the second sensor a second distance is measured between the second sensor and an outer surface of a second part of the wall that faces the second sensor, wherein from the first and second distance an outer diameter of the glass product is determined. Further, or instead, it can hold in particular that with the first sensor a third distance is measured between the first sensor and an inner surface of a first part of the wall that faces the first sensor and that with the second sensor a fourth distance is measured between the second sensor and an inner surface of a second part of the wall that faces the second sensor, wherein from the third and fourth distance an inner diameter of the glass product is determined. It is also possible that per glass product with the first sensor a first distance is measured between the first sensor and an outer surface of a first part of the wall that faces the first sensor and that with the second sensor a second distance is measured between the second sensor and an outer surface of a second part of the wall that faces the second sensor, wherein from the first and second distance determined for different products and associated rotational position an outer diameter and/or skew position of the virtual product is determined.

According to a special embodiment it holds that inspecting also comprises a controlling whereby at least one step of the steps a., b. and c. is adjusted on the basis of measuring results obtained using the at least one sensor and possibly the determined associated rotational positions, more particularly on the basis of the determined lateral glass thickness distribution of the virtual product. In this manner, the production process of the glass products can be strongly improved and optimized.

The method for producing and inspecting hollow glass products of glass product material is characterised in that inspecting the glass products comprises the following steps:
d. transporting the glass products formed in step b. successively along a predetermined path along at least one sensor for measuring a glass thickness, such as a Chromatic Confocal sensor, wherein with the at least one sensor, of a plurality of the glass products that are transported successively along the at least one sensor, per glass product at least one glass thickness is measured, wherein step d. is carried out between steps b. and c. and wherein in step d. of each glass product of the plurality of glass products on which measuring is done with the at least one sensor the rotational position of the glass product around an axial axis of the glass product relative to the at least one sensor is determined, wherein in a step e. the determined glass thicknesses and the associated rotational positions of the plurality of products are processed in combination for obtaining information about a lateral glass thickness distribution around an axial axis of a virtual glass product that represents the plurality of glass products.

The system for producing and inspecting hollow glass products is characterised in that the at least one sensor is a sensor for measuring a glass thickness such as a Chromatic Confocal sensor, wherein the system further comprises a rotational position unit, output signals of which are supplied to the signal processing unit for further processing, wherein the at least one sensor is set up such that the formed glass products that are supplied to the cooling unit, in use, are successively measured with the at least one sensor, wherein the rotational position unit is set up such that, in use, the rotational position of the glass product around an axial axis of the glass product relative to the at least one sensor is determined, wherein the signal processing unit is configured for, per scanned product, on the basis of the output signals of the at least one sensor and the rotational position unit, determining a lateral glass thickness distribution around an axial axis of a virtual glass product that represents the plurality of glass products.

The system for inspecting glass products is further characterised in that the system further comprises a rotational position unit such as a sensor, wherein the at least one sensor is a sensor for measuring a glass thickness such as a Chromatic Confocal sensor, wherein, in use, the at least one sensor is set up such that the glass products are successively measured with the at least one sensor, wherein the rotational position unit is set up such that, in use, the rotational position of the glass product around an axial axis of the glass product relative to the at least one sensor is determined, wherein the signal processing unit is configured for, per scanned product, on the basis of the output signals of the at least one sensor and the rotational position unit, determining a lateral glass thickness distribution around an axial axis of a virtual glass product that represents the plurality of glass products.

In practice, glass products are typically produced parallel to each other in a plurality of moulds.

According to the invention, per mould, for products that have been produced with that mould, the glass thickness distribution of at least one virtual product can then be determined. The glass thickness distribution of a virtual product determined on the basis of products produced with another mould is then determined separately. If one of the moulds exhibits a deviation resulting in a deviation in glass thickness distribution of an associated virtual product, this can be established separately for that mould. Also a deviation in at least one trough each time exclusively supplying a glass gob to one of the moulds, with such deviation resulting in a deviation in the glass thickness distribution of at least one virtual product determined on the basis of products produced from glass gobs that have flowed through the respective at least one trough, can be detected by detection of a deviation in the respective at least one glass thickness distribution. When deviations in troughs and/or moulds have thus been detected, these may be corrected, for instance by readjusting a position and/or orientation of a trough relative to a mould, and/or by providing a trough with a lubricant, and/or by replacing a mould, and/or by replacing a trough. This may be carried out automatically or manually.

Accordingly, it holds for the method in particular that a plurality of the steps b. are carried out parallel to each other for producing parallel to each other a plurality of the glass products in a plurality of production flows which each comprise a step b., wherein each glass thickness distribution of a virtual product has been obtained on the basis of measurements on glass products that have been manufactured in a same production flow, more particularly wherein on the basis of at least one determined glass thickness distribution of at least one virtual product that has been obtained from the measurements on glass products stemming from a same production flow, that production flow is controlled (automatically or manually). Controlling of a production flow is here understood to mean controlling of hardware with the aid of which in the production flow the product is manufactured. Such controlling can consist in, for instance, setting a position and/or orientation of at least one trough and/or a mould used in the respective production flow, supplying a lubricant to the at least one trough and/or replacing the respective mould and/or replacing the respective trough.

For the system, it holds in particular that production flows each comprise a step b., wherein the signal processing unit is configured such that, in use, each glass thickness distribution of a virtual product is obtained on the basis of measurements on products that have been manufactured in a same production flow.

More particularly, it holds furthermore for the system that on the basis of at least one determined glass thickness distribution of at least one virtual glass product that has been obtained from measurements on glass products stemming from a same production flow, that production flow is controlled in an automatic manner.

It holds accordingly for a method and system according to the invention that in particular the quality of each production flow is controlled separately. Per production flow, a lateral glass thickness distribution is then determined. If for instance in a production flow too great a deviation in a glass thickness distribution is then determined, an intervention in this production flow can be made automatically or manually, for instance by generating an alert related to the respective production flow or by readjusting the respective production flow or by replacing a mould being used in the respective production flow.

According to the invention, it holds that the sensor is not a passive infrared light sensitive sensor for obtaining an image of the product such as an infrared sensor. The at least one sensor for measuring the glass thickness may also, according to the invention, work according to the principle of laser interference. With laser interference, interference is measured between emitted laser radiation and the laser radiation received by reflection on the glass product and/or transmission by the object. The at least one sensor that is used according to the invention hence is an active sensor. An active sensor emits radiation and measures reflections of this radiation to determine a distance. The at least one sensor hence is not a passive sensor which only receives radiation, such as an IR camera. An active sensor is here also understood to encompass an assembly of which a first subsensor emits radiation and of which a second subsensor receives reflections on the glass product of the emitted radiation and/or transmissions by the glass product of the emitted radiation.

The invention will now be further explained on the basis of the drawing. In the drawing:
Figure 1A shows a possible embodiment of a system according to the invention for carrying out a method according to the invention;
Figure 1B shows a possible alternative embodiment of a system according to the invention for carrying out a method according to the invention;
Figure 2A shows the use of a part of the system of Figure 1A;
Figure 2B shows an alternative use of a part of the system of Figure 1A;
Figure 2C shows the use of a part of the system of Figure 1B;
Figure 3A shows a part of the use according to Figure 2A;
Figure 3B shows a part of the use according to Figure 2B;
Figure 3C shows a part of the use according to Figure 2C;
Figure 4 shows a possible implementation of the glass product that is manufactured in the system of Figures 1A and 1B;
Figure 5 shows schematically a 3D view of a glass product in which areas are hatched of which an LGD has been determined;
Figure 6 shows schematically a top plan view of a possible embodiment of the product forming apparatus of Figures 1A and 1B;
Figure 7 shows schematically a possible embodiment of the sensor of the system according to Figures 1A and 1B; and
Figure 8 shows schematically an alternative embodiment of the sensor of the system according to Figures 1A and 1B.

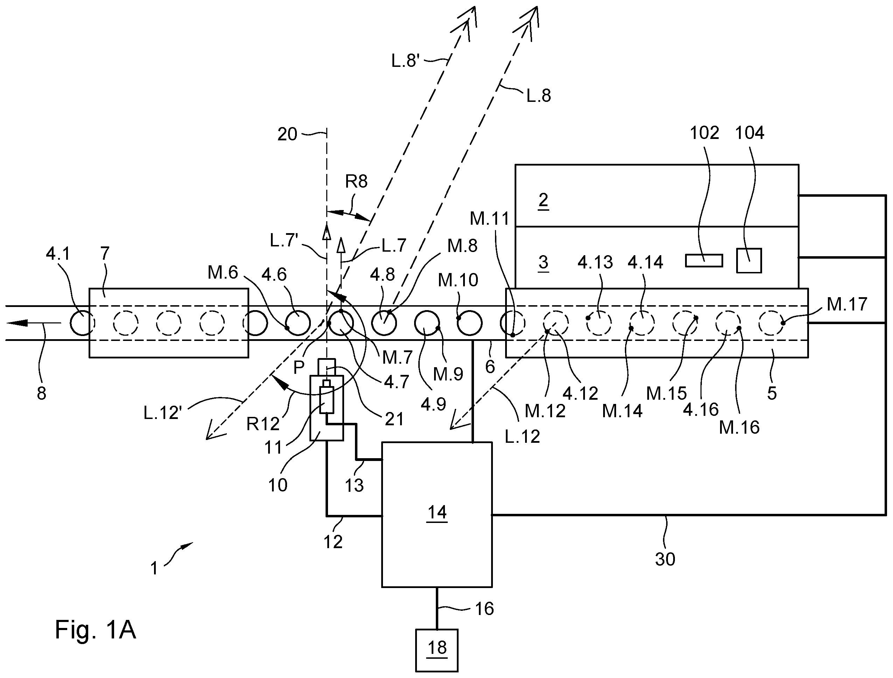

In Figure 1A there is indicated with reference numeral 1 a system according to the invention for carrying out a method according to the invention. The system comprises a schematically shown heating apparatus 2 for heating glass product material so that the glass product material enters a molten state. The molten material is transported to a product forming apparatus 3. In addition, other materials may be supplied to the product forming apparatus 3 if this is necessary, such as other materials and/or semimanufactures. In this embodiment, the product forming apparatus 3 successively manufactures in each case one hollow glass product 4.i (i=1,2,3,...) in a production flow. The glass product 4.i is here manufactured after the glass product 4.i-1. The product forming apparatus 3 in this example includes for this purpose one mould, known per se, 104 (shown schematically in Figure 1A) into which a portion of the heated glass product material is introduced, and at least one trough 102 (shown schematically in Figure 1A) to guide a glass gob to the mould (the glass gob slides via the inclined trough to the mould concerned). Also, the product forming apparatus 3 comprises blowing means (not shown) for blowing or pushing the glass product material into the mould for obtaining the final shape of the glass product. While in this example the product forming apparatus comprises one mould for manufacturing a glass product in one production flow, it is, of course, also possible that the product forming apparatus comprises a plurality of moulds for manufacturing parallel to each other a plurality of products in a plurality of parallel production flows. That example will, after the discussion of the variant with one mould, be discussed as well, with reference to a variant with six moulds.

The successively formed glass products 4.i are placed with the aid of a placing unit 5 on a conveyor 6. For different values of i, then, the respective products have been produced at different points of time because products are produced one at a time with one and the same mould in a single production flow.

The glass products 4.i produced as described above are transported using the conveyor 6 to a position P where inspection of a glass product can take place as will be set out hereinafter. Using the conveyor, the products are then transported further to a cooling apparatus 7 for cooling of the glass product. With the arrow 8, the direction of transport of the conveyor is indicated.

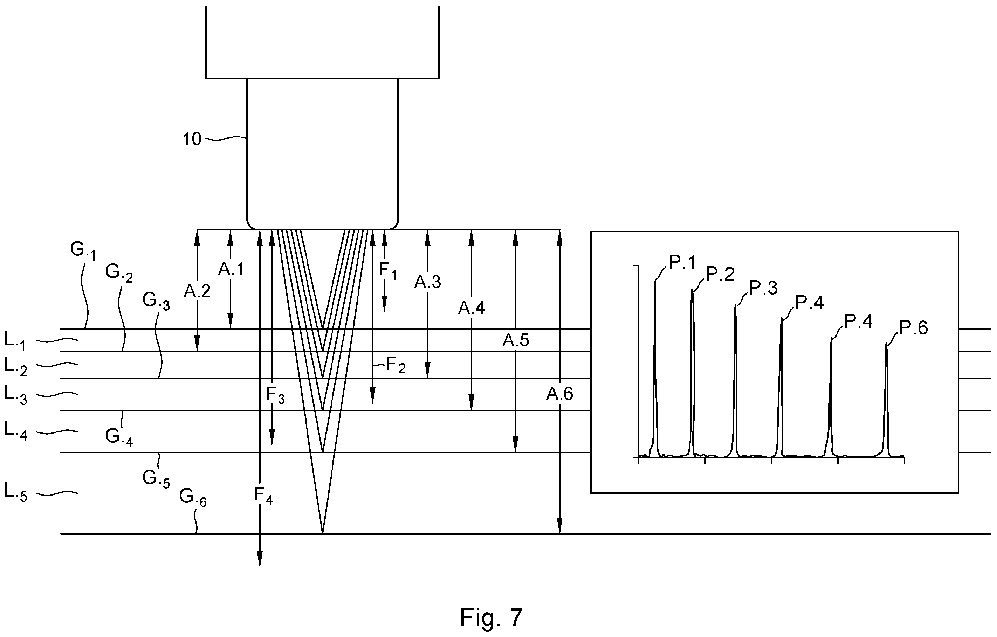

The system comprises at least one sensor 10 for measuring a glass thickness of a glass product 4.i when the respective glass product 4.i is at the position P. In this example the sensor 10 is a Chromatic Confocal sensor, known per se. The operative principle of the Chromatic Confocal sensor is shown in Figure 7. The sensor generates a beam of light with different colours. The focal distance F depends on the colour of the light. The beam comprises a plurality of colours, in particular a complete spectrum of white light. By way of example, in Figure 7, the focal distance for blue light of a particular frequency is indicated with F1. The focal distance for green light of a particular frequency is indicated with F2, the focal distance for yellow light of a particular frequency is indicated with F3 and the focal distance for violet light of a particular frequency is indicated with F4. In reality, there are infinitely many focal distances because the beam comprises all colours of visible light. In Figure 7, the light is incident on a transparent product with different glass layers stacked onto each other, L.l (l= 1, 2, 3..5). The light will reflect on the boundary surface G.g (g= 1, 2, 3, 4, 5, 6). Reflections occur on the outer surfaces of the layers L.l in respect of which it is assumed for simplicity that outer surfaces of different layers and which abut against each other coincide perfectly and thus form one single boundary surface. This has only been done to explain the working of the sensor and does not concern a limitation of the invention. At the boundary surface G.1, blue light of a particular frequency will reflect. Of this light, the focal distance coincides with the boundary surface G.1. The reflected light is received by the sensor 10 and the sensor issues a signal that represents the colour of light that is received. The receipt of this colour of light can be indicated in a frequency spectrum with a peak P.1. At the boundary surface G.2, green light of a particular frequency will reflect. Of this light, the focal distance coincides with the boundary surface G.2. The reflected light is received by the sensor 10 and the sensor issues a signal that represents the colour of light that is received. The receipt of this colour of light can be indicated in a frequency spectrum with a peak P.2. In general, it holds that at the boundary surface G.g light of a particular frequency will reflect. The reflected light is received by the sensor 10 and the sensor issues a signal that represents the colour of light that is received. The receipt of this colour of light can be indicated in the frequency spectrum with a peak P.g.

It will be clear that in the frequency spectrum, P.1 corresponds to the distance A.1 between the sensor 10 and the boundary surface G.1. It also holds that in the frequency spectrum, P.2 corresponds to the distance A.2 between the sensor 10 and the boundary surface G.2. More generally, it holds that in the frequency spectrum, P.g corresponds to the distance A.g between the sensor 10 and the boundary surface G.g. The thickness of layer L.1 then corresponds to the distance in the spectrum between peak P.1 and peak P.2. More generally, it holds that the distance between peak P.g and P.g+1 corresponds to the thickness of layer L.g. (g= 1,2,3,4,5,6). It will be clear that with the sensor 10 thus a wall thickness of a glass product 4.1 can be measured. Other types of sensors for measuring the wall thickness of the glass product can also be used as will be explained hereinafter.

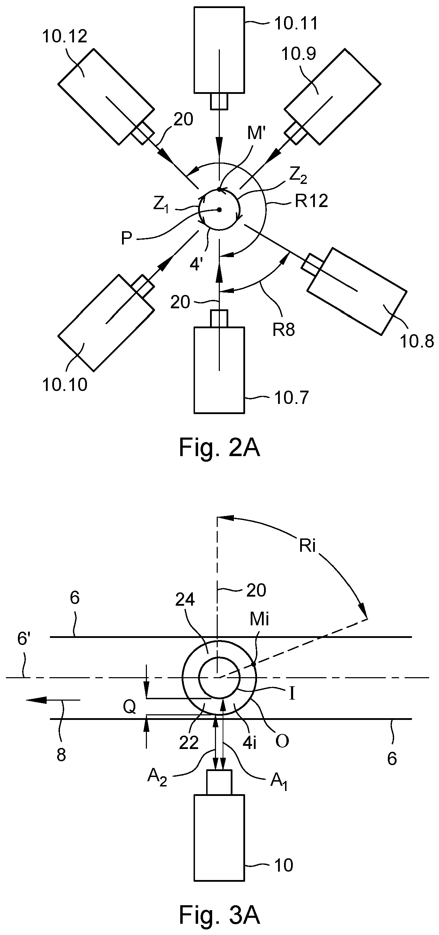

In this example, the system of Figure 1A furthermore comprises a rotational position unit 11 for determining a rotational position Ri of the product 4.i around an axial axis A of the product 4.i relative to the sensor 10. In this example, the rotational position unit comprises a camera 11 (which is placed on top of the sensor 10) for making a recording of the glass product 4.i when the respective glass product 4.i is at the position P. It holds in this example that with the sensor 10 a glass thickness of a glass product is measured when an axial axis of the respective glass product is at least substantially intersected by a line 20 which represents a measuring direction of the respective sensor. At the same time, also a recording of the respective glass product is made with the camera 11. Thus, it holds further that with the at least one sensor in each case a glass thickness is determined of a part Q of a wall of a glass product of which an outer surface O faces the at least one sensor (see Fig. 3A). Here, it holds further that with the at least one sensor a first distance A1 is measured between the at least one sensor 10 and the outer surface O of the part of the wall and a second distance A2 is measured between an inner surface I of the part of the wall, wherein from the first distance and the second distance the glass thickness of the part Q of the wall is determined.

Via line 12 which is connected with the sensor 10, a signal of the sensor is supplied to a signal processing unit 14. Via line 13 which is connected with the camera 11, a signal of the camera 11 that represents the recording is supplied to the signal processing unit 14. The signal processing unit 14 is connected via a line 16 with a display 18.

The working of the system according to the invention is as follows. In a step a. glass product material is heated with the heating unit 2. Then, in a step b. with a product forming apparatus the heated and molten 'liquid' glass material (often having the shape of a gob then) is formed into a glass product 4.i. In a step c. the glass product is cooled with a cooling apparatus 7.

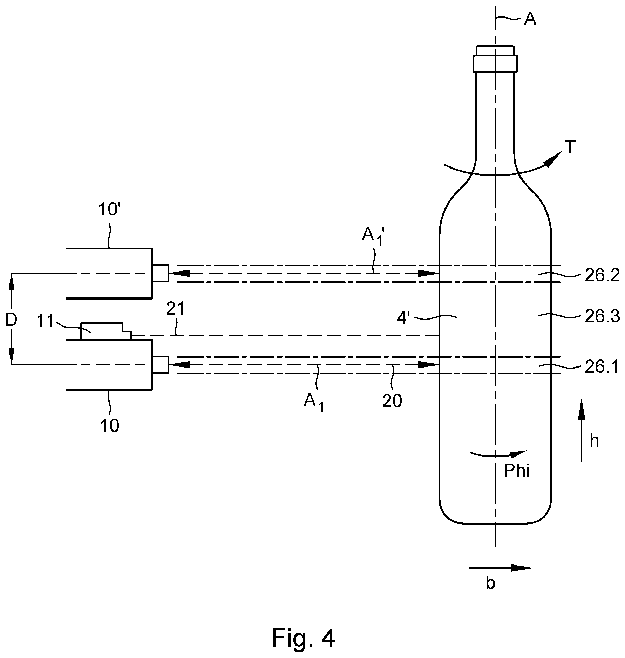

The glass product 4.i formed in the product forming apparatus is placed with the aid of the placing unit 5 on the conveyor 6 for transport in the direction 8. Glass product 4.i, as has been mentioned, is produced after glass product 4.i-1. The glass products in this case are bottles as shown in Figs. 4 and 5. The glass product 4.i is provided with an axial axis A which in this example is vertically directed. If a product 4.i which has been formed arrives at the position P, a glass thickness measurement is performed on the product with the sensor 10.

Further, it holds in this example that with the aid of the signal processing unit 14, from the image that is made of the product 4.i with the camera 11, the rotational position R.i of the glass product 4.i on the conveyor around its axial axis relative to, in this example, the measuring direction 20 of the sensor is determined. This may for instance be carried out by, in an image made with the camera, detecting (with for instance pattern recognition technology) where a marking and/or a seam and/or a dot M of the glass product is. Leaving the difference in height aside, it holds that the measuring direction 20 of the sensor is equal to the direction of the optical axis / viewing direction 21 (see Fig. 4) of the camera 11.

Because the recording with the camera 11 thus already comprises information about the rotational position R.i of the glass product 4.i that is visible on the recording, it is stated that the rotational position is determined in step d. Because the rotational position is recognized by the signal processing unit from a recording, it may also be said that the rotational position has been determined in step e. The rotational position Ri of the glass product 4.i may then for instance be an angle Ri with respect to the measuring direction 20 (see Figures 1A and 3A). For when the glass product is manufactured, it may beforehand be provided with a marking such as a dot or a seam. When the glass product is placed on a conveyor with the aid of the placing means 5, knowledge about the rotational position of the product is lost because during placing the product can turn about its axial axis. It holds accordingly that on the conveyor each product has a more or less random rotation direction around an axial axis of the respective product. Each product that is placed on the conveyor accordingly has a rotational position varying per product around an axial axis of the product. It holds accordingly that the glass products between steps b. and c. are transported on a conveyor along the path, wherein in particular each glass product between steps b. and c. is placed on the conveyor with a rotational position varying per glass product around an axial axis of the glass product. Further, it holds accordingly, in this example, that the glass product between steps b. and c. is transported on the conveyor from a product forming apparatus such as a mould in which the glass product has been formed in step b. to a cooling apparatus in which the product is cooled in step c. Also, it holds accordingly in this example that the rotational position of each of the glass products of the plurality of glass products around its axial axis on the conveyor is determined. Further, it holds accordingly in this example that using camera 11 the rotational position of a glass product of the plurality of glass products is determined by recognizing a predetermined marking (such as a seam or a dot) in an image of the respective product that has been made with the camera 11.

Thus, therefore, on each product 4.i arriving at the position P, at least one glass thickness measurement is performed with the sensor 10, the information of which is supplied via line 12 to the signal processing unit 14. Also, of each product 4.i the recording that has been made with the camera 11 is supplied via line 13 to the signal processing unit 14.

The signal processing unit determines from the information about the image the rotational position R.i of the respective product 4.i. Of course, in step d. the rotational position may also be determined in a different way, for instance with a different type of sensor 11 whose signals that contain information about the rotational position are supplied to the signal processing unit.

In Figure 1A, with M.i (i =.1,2,3,..), respectively, the marking of the glass product 4.i (i = 1,2,3,..) is indicated. As can be seen from the markings M.i, the glass products have a random rotational position with respect to the centerline 6' of the conveyor 6 and hence a random rotational position R.i with respect to the measuring direction 20. This in contrast to the product forming apparatus 3 in which all products 4.i, as soon as they have been formed, have a same rotational orientation with respect to the centerline 6'. This rotational position, however, as mentioned, can change when a product 4.i is placed on the conveyor.

Due to the products 4.i having a random rotational position Ri on the conveyor 6, the successively performed measurements on the products 4.i will in each case represent a glass thickness of the products relative to the marking Mi from a different measuring direction. For simplicity, it is assumed that per product one wall thickness measurement is performed (later an example will be given of a variant where per product a plurality of thickness measurements are performed on the product at different positions). If it is assumed that all products are the same, there will be available, given a sufficient number of measurements, a set of wall thicknesses that are spread more or less regularly in tangential direction over a complete revolution around the axial axis of the product.

If there is a fault or a faulty setting present in for instance the glass product forming apparatus, it will typically be present in all glass products 4.i at the same position with respect to the marking M.i. By analysing sufficient thickness measurements on the successively made products 4.i, a fault or deviation can be found in the whole first area. Accordingly, a fully circular course of the wall thickness of a virtual product 4' can be constructed from a sufficient number of measurements on the products 4.i. Here, use can be made of the known specifications of dimensions (including shape, size and wall thicknesses) of the products on which measuring is done. Hence, also the shape and size of the virtual product are known. In this virtual product, the wall thicknesses measured on real products may then be drawn in or be noted in a table on the basis of the wall thicknesses and associated rotational positions measured on different real products. To put it differently, the specified measurements (including wall thicknesses) of the product are adopted for the virtual product and corrected on the basis of the measurements, there where the measurements deviate from the specifications. It holds, accordingly, that on a sufficiently large number of products from a production flow measurements are performed with the at least one sensor 10 in order that these measurements in combination cover the associated virtual product around the axial axis A of the virtual product with mutually neighbouring distances in tangential direction that are each smaller than a predetermined value. The direction of the axial axis of a product 4.i is the same as the axial axis of the virtual product 4'.

It holds here that in step e. the lateral glass thickness distribution is determined in a first area 26.1 (see Figure 4) of the virtual glass product that extends around the axial axis of the virtual glass product.

This is further clarified in Figure 2A. In Figure 2A, with sensor 10.8 the relative virtual position of the sensor 10.8 is indicated with respect to the glass product 4.8 when at the position P a thickness measurement is performed on the glass product 4.8. Here, in Figure 2A, the position and orientation of the glass product 4.8 has been chosen such that M.8 coincides with a randomly fixedly chosen position M' (in this example fixedly chosen to be 'at twelve o'clock' in the drawing). Also, with sensor 10.12, the relative virtual position of the sensor 10.12 with respect to the glass product 4.12 is indicated, when at the position P a thickness measurement is performed on the glass product 4.12. Here, in Figure 2A, the relative position of the product 4.12 has again been chosen such that M.12 coincides with the randomly fixedly chosen position M'. In Figure 2A, thus, the rotational position of the glass product 4.8 is imaged so as to be coincidental with the rotational position of the product 4.12, such that M.8 coincides with M.12. Looking at the real position of the sensor 10 with respect to the glass product 4.12 when the glass product 4.12 is at the position P, the measuring direction 20 of the sensor 10 and the rotational position of the product 4.12 measured with respect to the marking M.12 include an angle R.12 (see Figure 1A). The rotational position of the product 4.12 is indicated in Figure 1A with the line L12. The line L12' is parallel to the line L12 and indicates the rotational position of the product 4.12 when it would be at the position P. In that position, the rotational position of the product 4.12 with respect to the measuring direction 20 of the sensor can be indicated with the angle R.12. In Figure 2A the same rotational position R.12 is indicated. However, because in Figure 2A the rotational position of the product 4.12 is imaged so as to be coincidental with the rotational position of the product 4.8 (since M.8 and M.12 coincide in M' in Figure 2A), the virtual position of the sensor 10.12 will not coincide with the virtual position of the sensor 10.8. In Figure 2A, entirely analogously, for the products 4.7, 4.9, 4.10 and 4.11, the virtual positions of the associated sensors 10.7, 10.9, 10.10 and 10.11 are indicated. The measurements of the virtual sensors 10-7 - 10-12 in Figure 2A form in combination a measuring range that extends around a virtual product 4'. The position of the sensor 10.i in Figure 2A can accordingly be obtained by rotating the product 4.i and the sensor 10 from Figure 1A such that M.i coincides with M'. To put it differently, a measurement with the virtual sensor 10.i on the virtual product 4' in Figure 2A is equivalent to a measurement with the real sensor 10 on product 4.i in Figure 1A.

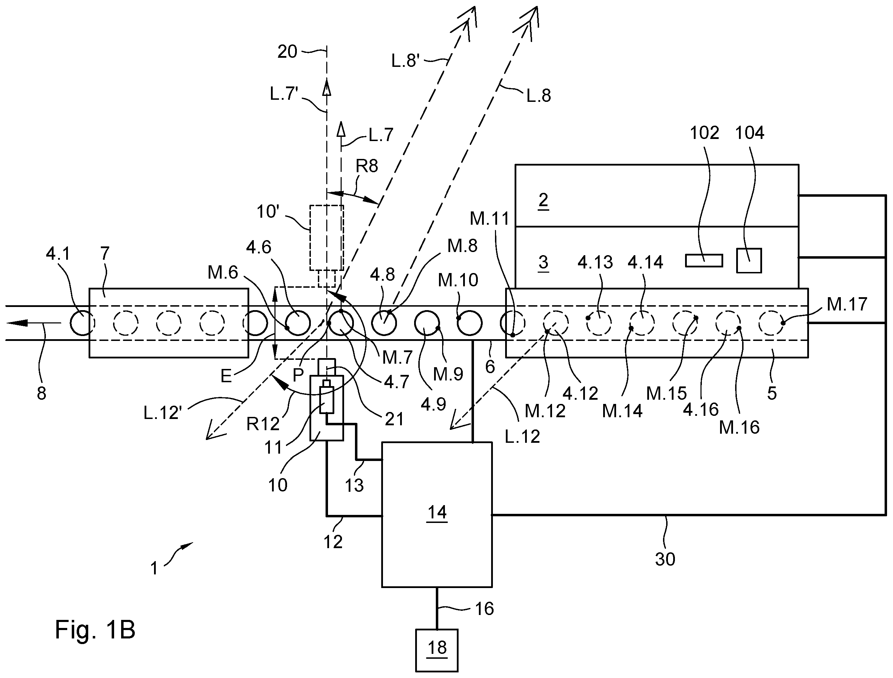

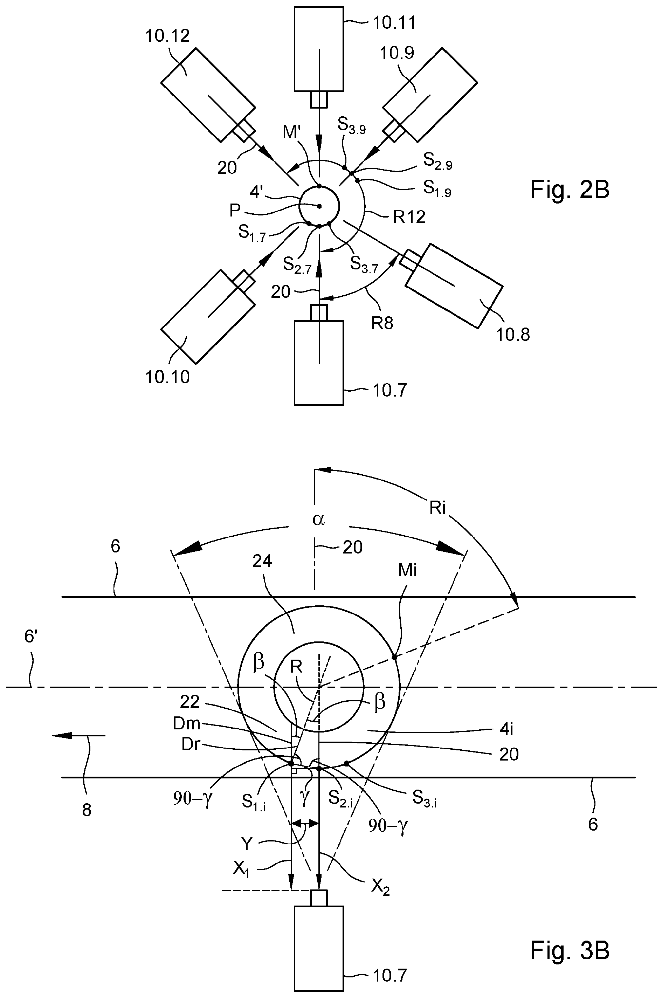

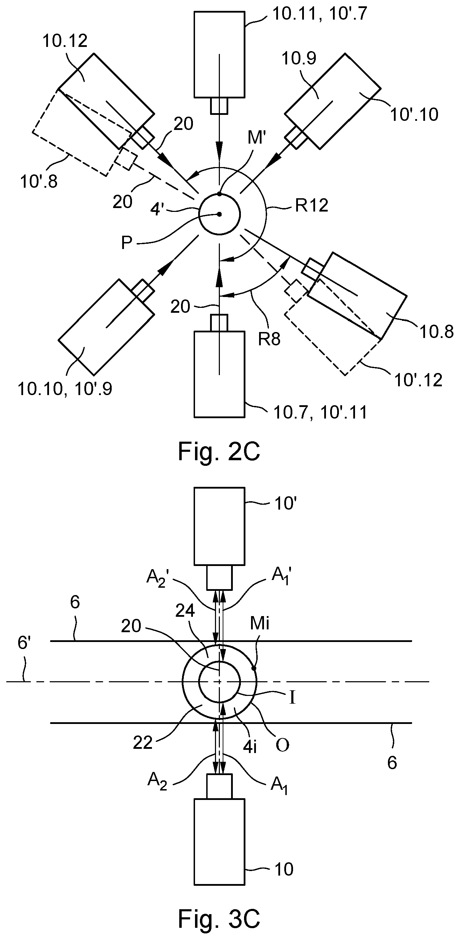

In Figure 2A the position of the virtual sensor 10.12 has therefore been chosen such that it has the rotational position R.12 with the virtual product 4' with marking M'. To put it differently: in Figure 2A the product 4.12 and the sensor 10 have been rotated around the axial axis of the product 4.12 at the position P such that the marking M.12 of the product 4.12 also coincides with the marking M' of the virtual product. In that regard, in Figure 2A, after rotation the virtual position of the sensor 10 for product 4.12 is indicated with 10.12 and after rotation the virtual position of the sensor 10 for product 4.8 is indicated with 10.8. This has been done in Figure 2 entirely analogously for the products 4.7, 4.9, 4.10 and 4.11. For instance, entirely analogously, in Figure 1, the rotational position of the product 4.11 is indicated with the line L11. The line L11' is parallel to the line L11 and indicates the rotational position R.11 = 0 of the product 4.11 when it would be at the position P. In that position, the rotational position of the product 4.11 with respect to the measuring direction 20 of the sensor 10 can be indicated with the angle R11. In Figure 3A the rotational position R.i for the random product 4.i is indicated.

In this example, it holds that when in each case respective measurements on six successive products are combined, on the virtual product the positions at which the glass thickness has been determined are located reasonably distributed around the axial axis of the virtual product, see for example Figure 2. The wall thicknesses at these positions in combination provide information about a lateral glass thickness distribution in a first area 26.1 (see Figure 4 for the areas on the products 4.i) of the virtual product 4' (see Figure 5 for the areas of the virtual product 4'). Of course, this is a static process where the distribution can vary when in each case six measurements based on six real glass products are used for obtaining information about a lateral glass thickness distribution. This process can subsequently be repeated for the products 4.13-4.18 for again determining the lateral glass thickness distribution in the area 26.1. In this manner, any drift in time (trend) of the lateral glass thickness distribution can be detected. If a trend in a deviation of successively determined lateral glass thickness distributions is determined, it may for instance be inferred that a particular component, such as a mould, is wearing.

It may also be chosen, of course, in each case to combine eight measurements based on eight real products for obtaining information about a lateral glass thickness distribution of a virtual product. Also, it is conceivable that in each case on a sufficient number of products from a production flow measurements with the at least one sensor are performed in order that these measurements in combination cover the associated virtual product around the axial axis of the virtual product with mutual neighbouring distances (neighbouring distance between measuring positions) in tangential direction that are each smaller than a predetermined value. For clarity's sake, such a mutual distance between two neighbouring measurements is indicated in Figure 2A with Z1. In Figure 2A, accordingly, six of such distances can be defined. Z2 is a second neighbouring distance that can be defined, etc. In each case, a glass thickness measurement is performed on a new product that is present at the position P, until the set of all measurements performed until that moment is so large that in combination they cover the associated virtual product around the axial axis of the virtual product with mutual neighbouring distances in tangential direction that are each smaller than a predetermined value. Z is thus smaller than the predetermined value, then. This may then take place, as the case may be, upon for instance 20 measurements so that 20 neighbouring distances have been formed which are each smaller than the predetermined value. After this, a new measuring series on successive products is started until this set of these thickness measurements is sufficiently large for the measurements of this set to cover the associated virtual product around the axial axis of the virtual product with mutual neighbouring distances in tangential direction that are each smaller than a predetermined value. Because this methodology is performed repeatedly in time, also a good impression of the course of a lateral glass thickness distribution in time can be obtained.

It will be clear that performing the plurality of measurements in step d. is carried out between the steps b. and c.

The result of each measurement that is made with the sensor 10 is respectively supplied via line 12 to the signal processing unit 14. In a step e. these signals, together with the associated information about the rotational positions, are processed in combination for obtaining the information about the lateral glass thickness distribution around an axial axis of the virtual glass product 4' which represents the plurality of glass products 4.i as discussed above.

In particular, it holds that the glass thickness distribution indicates relative variations in glass thickness. Also, it is possible that the glass thickness distribution comprises absolute values of the glass thickness distribution. Further, it is possible that the lateral glass thickness distribution is depicted on the display 18 in a 3D picture of the product 4'. To this end, for instance, in the signal processing unit information is stored about the dimensions (including shape and wall thickness) of the product to be produced. This is also referred to as the product specifications. Ideally, a produced product has exactly the same dimensions as specified. However, the lateral glass thickness distribution determined with the aid of the sensor in the first area may deviate therefrom. The determined glass thickness distribution can now be added to an image of the specified product for obtaining the 3D picture of the virtual product 4'. This virtual product then shows in the first area the measured wall thicknesses. While the positions on the virtual product to which the measured wall thicknesses relate are separate from each other in tangential direction, the wall thickness that is shown in the 3D picture may be a wall thickness that runs gradually and connects the actually measured wall thicknesses with each other (similarly to drawing a flowing line through a number of measuring points in a graph). The information that has been obtained about a lateral glass thickness distribution, however, may also be depicted on the display in a table.

In the case discussed, with the sensor 10 measuring is done in a first area 26.1 (see Figure 4) in which with the signal processing unit the LGD is determined (for instance the LGD at half height of that area), see Figure 5, where the half height of area 26.1 is indicated with a dotted line. In that case, using a second sensor 10' which is at a greater height h than the sensor 10 and whose output signals are also supplied to the signal processing unit (not shown in the drawing), a lateral glass thickness distribution in a second area 26.2 extending around the product 4' can be determined with the signal processing unit (see Fig. 5). The second area 26.2 is staggered with respect to the area 26.1 in axial direction (see Figure 5). The LGD in the second area 26.2 is for instance also at half height of this area (indicated by a dotted line in Figure 5). Also, entirely analogously, it is possible that using a third sensor (shown in Figure 4, not in Figure 5) the lateral glass thickness distribution in an area 26.3 is determined. The height of an area 26.1, 26.2 and 26.3 has for instance been chosen such that the glass thickness substantially does not change in vertical direction (= axial direction A). The glass thickness within such an area will then, if it changes, vary especially in tangential direction. The areas 26.1, 26.2 and 26.3 for instance adjoin each other. It holds thus for the areas 26.1 and 26.2 that steps d. and e. are carried out repeatedly for obtaining a lateral glass distribution LGD in the first and second area of the glass product extending around an axial axis of the product, with the first and second area being staggered with respect to each other in the axial direction A.

In particular, it holds for the areas 26.1 to 26.3 that the steps d. and e., respectively, are carried out repeatedly at least three times for respectively obtaining lateral glass thickness distributions in respectively at least three mutually different areas which each extend around the axial axis and are staggered with respect to each other in axial direction and which preferably in combination cover at least substantially the whole glass product 4'. It is also possible that the lateral glass thickness distribution in different areas such as the areas 26.1 and 26.2 are determined with one and the same sensor 10. To this end, the sensor 10 in the system of Figure 1A needs to be set up in a height-adjustable manner so that per product measurements can be made at different heights, or measurements can be made on a number of successive products at a first height, on a different number of successive products at a second height, etc. Thus, it is also possible that the sensor 10, after measuring the wall thickness for obtaining a glass thickness distribution in the area 26.1, is moved upwards in axial direction for performing a measurement to obtain a lateral glass thickness distribution in the area 26.2. In that case, the sensor 10' is not present and the sensor 10 can be moved up for measuring in the area 26.2 after the sensor 10 has measured in the area 26.1. After this, the sensor can be moved downwards for carrying out a measurement in the area 26.3, etc.

In the above-mentioned manner, the lateral glass distribution LGD of the virtual product has been determined as a function of h and phi. Here, h is defined by the height at which the sensor 10 in Figure 1A is set up. This will be further explained with reference to Figures 4 and 5. If LGD as a function of particular values of h and phi is known, a continuous function LGD (h, phi) can be determined which, for the determined values of h and phi, takes the measured glass thickness. The glass distribution LGD (h,phi) can via a line 16 be shown on the display 18, for instance by means of numbers, a graph or an image. LGD is then for instance a number that indicates the absolute wall thickness at the position (h,phi). It is also possible, however, that LGD indicates a relative wall thickness with respect to a reference wall thickness at the position (h, phi). Also, the lateral wall thickness may be shown in a 3D picture of the glass product which is depicted on the display. This picture then shows, for instance, a view of the glass product in transparent view. In this way, an operator can keep an eye on the glass thickness distribution of the successively produced products 4.i. When the glass thickness distribution starts to deviate, for instance because the wall thickness of the product is becoming too large or too small at certain points, an intervention can be made in the production process, that is, an intervention can be made in the method step b. Such an intervention can be performed manually. However, it is also possible for such an intervention to be performed automatically with the aid of the signal processing unit, in this case via a feedback control line 30. It is possible, in case of a deviation of a glass thickness distribution, to correct manually or automatically in the production process of the products. It is also possible, in case of a change per unit time in glass thickness distributions successively obtained spread in time and the change exceeding a predetermined value, to correct or control manually or automatically under control of the signal processing unit via line 30. Correcting or controlling may for instance be understood to mean adjusting a position and/or orientation of a trough and/or mould, providing a trough with a lubricant and/or replacing a mould. All this is schematically indicated in Figure 1A.

Further, it is possible that a calibration measurement is performed on a glass product having a known glass thickness, on the basis of which thereupon step e. is carried out. For if the glass thickness is known upon the calibration measurement, the signal processing unit 14 can correct the lateral glass thickness distribution of the product 4'.

The invention is not in any way limited to the embodiments outlined above.

For instance, it is clear that the sensor 10 and the signal processing unit 14 can also be used in other production processes for forming glass products than described here. In fact, the sensor in combination with the signal processing unit 14 constitutes an essential part of the invention. According to the invention, also, the cooling apparatus 7 could be omitted, since also without cooling apparatus 7 the products will eventually cool down as a matter of course so that step c. may also be carried out without extra aids. Also, the cooling apparatus may, whether manually or automatically, be controlled (for example, the temperature of the cooling apparatus) on the basis of the determined LGD.

In this example, a lateral glass thickness distribution of the virtual product 4' has been obtained from measurements on the products 4.7-4.12. As mentioned, after this, for instance on the basis of the products 4.13-4.18 a next lateral glass thickness distribution of the virtual product 4' can be obtained entirely analogously to what has been discussed hereinabove for the products 4.7-4.12. This can be seen as an update in time of the current lateral glass thickness distribution.

More generally, from m measurements on the products 4.1+k with k=0, 1,2,3,...m-1, the glass thickness distribution of a virtual product can be obtained. Next, from m measurements on the products 4.1+m+k with k=0, 1,2,3,...m-1, a glass thickness distribution of a virtual product can be obtained. After this, from m measurements on the products 4.1+2m+k with k=0, 1,2,3,...m-1, a glass thickness distribution of a virtual product can be obtained and then from m measurements on the products 4.1+3m+k with k=0, 1,2,3,...m-1, a glass thickness distribution of a virtual product can be obtained. This can be continued so that after each m measurements a glass thickness distribution of a virtual product is determined. This brings with it that changes of the glass thickness distribution in time can be observed. This makes it possible to detect changes in the glass thickness distribution of successively determined virtual products as a result of for instance wear in troughs and the mould, or changes in the glass thickness distribution of successive virtual products as a result of drifting settings of the trough and/or mould. When such a change exceeds a limiting value, automatically or manually the production flow may be controlled (as by setting a position and/or orientation of a trough and/or mould, supplying a lubricant to a trough, replacing a mould, etc.).

It is also possible that m is variable. In that case, generally, according to the invention (apart from the embodiments) the signal processing unit is configured to select a sufficient number of successive measurements in order that the virtual sensors (Figure 2A) associated with these measurements cover the associated virtual product completely around the axial axis of the virtual product with mutual neighbouring distances measured in angles in tangential direction which are each smaller than a predetermined angular value. This distance may for instance be 1/20 part of the circumference so that an accurate course of the LGD is obtained.

The signal processing unit then decides automatically which and how many measurements in each case are processed in combination for computing a glass thickness distribution of a virtual product.

In particular, it holds that in step d. with the at least one sensor 10 successively a plurality of wall thicknesses are determined of a glass product which is transported along the at least one sensor so that the determined glass thicknesses relate to different positions of a wall of the glass product, these positions being separate from each other in a direction in which the product is transported in step d. This provides the advantage that with the at least one sensor 10 glass thicknesses are determined at a plurality of positions which are separate in tangential direction of the product. Suppose that this is about three measurements in each case here, as shown in Figures 2B and 3B. In Figures 2A and 2B mutually corresponding parts are provided with a same reference numeral. Also in Figures 3A and 3B, mutually corresponding parts are provided with a same reference numeral. In Figure 3B this means that at the positions S1.i, S2.i and S3.i of product 4.i the wall thicknesses are measured. This is because, as 4.i is transported in the direction 8, with the sensor 10 successively glass thickness measurements are performed at the positions S1.i, S2.i and S3.i, respectively. In Figure 3B the positions S1.i coincide for all values of i (so for all glass products 4.i on which measurements are made). Also, in Figure 3B the positions S2.i coincide for all values of i. Further, in Figure 3B the positions S3.i coincide for all values of i. In Figure 2B the positions S1.i for all values of i (here i = 7,8,9,10,11,12), by contrast, do not coincide. This holds, respectively, also for the relative positions S2.i and the relative positions S3.2. In Figure 2B this would then mean that on product 4.7 not one measurement with the virtual sensor 10.7 has been performed as in Figure 2A, but three measurements. Also with the virtual product 4', the positions of the three measurements S1.7, S2.7, S3.7 on the product 4.7 are separate from each other in tangential direction of the glass product 4.i by a same mutual distance as in Figure 3B. The processing of the thickness measurements S1.7 and S3.7 is thus equal to the processing of the thickness measurement S2.7 as has already been described hereinbefore with reference to Figures 2A and 3A. Entirely analogously, in this example, each assembly of three measurements on a random product 4.i yields three measurements for the virtual product 4'. As an example, the measurements on product 4.9 are shown in Figure 2B with S1.9, S2.9 en S3.9.

For that matter, the wall thickness Dm which is measured at position S1.i (see Figure 3B) is equal to the wall thickness measured in the measuring direction 20. This thickness is somewhat greater than the wall thickness Dr that would be measured at the position S1.i in a radial direction R (= the direction of the normal to the outer surface of the glass product at the location of the position S1.i) at the position S1.i. In practice, this concerns only a small deviation when the radial direction mentioned includes a small angle β (for instance, smaller than five degrees) with the measuring direction 20. Of course, the wall thickness Dm measured with the sensor 10 can also be automatically corrected (converted into Dr) by the signal processing unit when the angle β is known. The system can in particular automatically determine or estimate this angle. For instance, the angle β may be estimated from a measured distance X2 between the sensor 10 and an outer surface of the glass product at position S2.i and the measured distance X1 between the sensor 10 and the outer surface of the sensor at position S1.i. In Figure 3B the measuring positions S1.i and S2.i are connected with a straight line as an approximation for the course of the outer surface. This straight line includes an angle y with a line perpendicular to the measuring direction. Also, this line includes an angle 90-y with the measuring direction. The angle β between the measuring direction and the line R is part of an isosceles triangle which comprises two equal angles 90-y. It follows that β= 2y. The arctan of X1-X2/ Y (where Y is the distance over which the glass product has been transported in the transport direction 8 between the measurement at position S1.i and the measurement at position S2.i) then equals y where y is an estimate for β/2 (see also Fig. 3B). Since X1, X2 and Y are known, the angle β can be estimated therefrom. The thickness Dr in radial direction at position S2.i is then equal to the measured thickness Dm times the cosine of the respective estimated angle for βa. This holds entirely analogously for the measurement at S3.i. Here too, the measurement can be corrected, in this example also with the cosine of the estimated angle for β. The thickness measured at the position S2.i does not need correction, for the angle β equals zero here. Of course, there are other possibilities of determining and/or estimating the angle β. Further, the angle β or an estimation therefor can be determined only once if the positions S1.i, S2.i and S3.i are fixed and are the same for each glass product 4.i on which measurements are made.

All of this holds for the measurements regarding the products 4.7-4.12 so that regarding the virtual product of Figure 2B in effect 3*6=18 glass thickness measurements are available which are separate from each other in tangential direction of the virtual product 4' and which are used to be able to determine the lateral glass thickness more accurately than in the method according to Figures 2A and 3A. The chance that in these 18 glass thickness measurements the distance in tangential direction between neighbouring measurements is smaller than the earlier-mentioned predetermined value is thereby increased. In practice, for instance 30 successive glass thicknesses may be determined regarding a single product as has already been discussed hereinabove for three successive glass thicknesses.

As already mentioned, it holds in particular that in step d. the product is transported in a horizontal plane, with the at least one sensor being moved in vertical direction for measuring on the glass product at different heights. This makes it possible that at least one glass thickness of the product is determined at different heights.

If at different heights a glass thickness is determined, it is also possible that from the measuring results obtained with the at least one sensor a skew position of the product 4.i is determined. In Figure 4 an example is given where a first sensor 10 and a second sensor 10' are worked with. With the first sensor 10 the distance A1 is measured between the sensor 10 and an outer surface of the product 4.i. With the second sensor 10' a distance A1' is measured between the outer surface of the product 4.i and the second sensor 10'. The difference in height between the sensors 10 and 10' is D. From the difference between the measured distances A1-A1' and the associated difference in heights D, the skew position of the product 4.i can be determined. It is also possible that the distance A1' is measured with the first sensor 10 by moving it in vertical direction to the position of the second sensor in Figure 4. The second sensor 10' is omitted then.

It is further possible that in step d. use is made of at least two mutually opposite and mutually facing sensors, with the products being transported between the at least two sensors. Per sensor 10, 10' the same measurements are then performed as discussed hereinbefore in the context of Figure 1A. In Figure B an example is given in which the sensors 10 and 10' are used which are located opposite to each other. In Figures 1A and 1B mutually corresponding parts are provided with a same reference numeral. The corresponding positions of the virtual sensors 10.i and 10'.i with respect to the virtual product are shown in Figure 2C. Here, 10.i are the virtual sensors that are associated with the sensor 10 of Figure 1B in respect of the glass product 4.i, and 10'.i are the virtual sensors that are associated with the sensor 10' of Figure 1B in respect of the glass product 4.i. For instance for product 4.8, it holds that the virtual sensor 10.8 with respect to the virtual product 4' (corresponding to the sensor 10 with respect to the product 4.8) is at the position indicated. The position of M' has here been chosen the same as in Figure 2A. For instance for the product 4.8, it holds further that the virtual sensor 10'.8 with respect to the virtual product 4' (corresponding to the sensor 10' with respect to the product 4.8) is at the position as indicated. In general, it holds that in Figure 2C the positions of the virtual sensors 10.7-10.12 correspond to those of Figure 2A and that the position of the virtual sensor 10'.i is opposite to the position of the virtual sensor 10.i for i=7-12. By chance, the position of the virtual sensor 10.11 thereby coincides with the position of the virtual sensor 10'.7. Entirely analogously, by chance, the positions of the virtual sensors 10.10 and 10'.9, 10.7 and 10'.11, 10.9 and 10'.10 respectively coincide. Accordingly, at the positions where the positions of virtual sensors coincide, there are two measuring values for the glass thickness. These may be averaged. At positions where the positions of the virtual sensors do not coincide, there is, with respect to the system of Figure 1A, an extra measuring value at a position that is new with respect to the system of Figure 1. So, this gives two extra measuring values for the determination of a lateral glass thickness, with the mutual distance between each extra measuring value and its neighbouring measuring values being smaller than the mutual distance between the neighbouring measuring values concerned. To put it differently, an average mutual distance between neighbouring measuring values decreases due to extra measuring values that do not coincide with the original measuring values. Accordingly, this provides a better insight on a lateral glass thickness distribution.

All measuring values can be processed in combination as discussed for Figure 1. It holds, then, that with the sensor 10 of the at least two sensors a first glass thickness of a first part of the wall of the glass product is determined and with the second sensor 10' of the at least two sensors a second glass thickness of a second part of the wall of the glass product is determined, wherein the first part and the second part are located on mutually opposite sides of the glass product. Further, it holds in particular (see Figure 3C) that with the first sensor 10 a first distance A1 is measured between the first sensor and an outer surface O of a first part of the wall that faces the first sensor and that with the second sensor 10' a second distance A1' is measured between the second sensor 10' and an outer surface O of a second part of the wall that faces the second sensor, wherein from the first and second distance an outer diameter of the glass product is determined by the signal processing unit 14. This outer diameter equals E-A1-A1' where E is the distance between sensors 10 and 10'. Further, it holds in this example that with the first sensor 10 a third distance A2 is measured between the first sensor 10 and an inner surface I of a first part of the wall that faces the sensor 10 and that with the second sensor 10' a fourth distance A2' is measured between the second sensor and an inner surface I of a second part of the wall that faces the second sensor 10', wherein by the signal processing unit 14 from the third and fourth distance an inner diameter of the glass product is determined. This inner diameter equals E-A2-A2' where E is the distance between the sensors 10 and 10'. The determined inner and/or outer diameter may, per product, be depicted on the display 18. Also, as an alternative, for instance, a current average of the last x products produced may be computed by the signal processing unit and be depicted on the display 18, where x is a integer greater than 1.

In particular, it holds further that, per product, with the first sensor 10 a first distance A1 is measured between the first sensor and the outer surface O of a first part of the wall that faces the first sensor 10 and that with the second sensor 10' a second distance A1' is measured between the second sensor 10' and an outer surface O of a second part of the wall that faces the second sensor 10', wherein from the first and second distance A1 and A2 and associated rotational position determined for different products an outer diameter and/or skew position of the virtual product is determined. Thus, an outer diameter B.i can be determined of the virtual product from the distance A1.i and A1'.i associated with the sensors 10.i and 10'.i from Figure 2C. This outer diameter is E-A1.i-A1'.i. In this way, six outer diameters are determined that have respectively been obtained on the basis of the products 4.i (i= 7,8,..12). This means that the outer diameter of the virtual product can vary in tangential direction. In this example, with the virtual product the position of the outer diameters B.7 and B.11, B.10 and B.9, B.8 and B.12 coincides. These pairs can be averaged for a better estimate of the outer diameter of the virtual product at the respective position. It is thus possible that the outer diameter is determined for the virtual product depending on a tangential position phi. This is because the outer diameter for different tangential positions on the outer surface of the virtual product corresponds to the outer diameter that has been determined in real products whose rotational position corresponds to a tangential position with the virtual product. Thus, the outer diameter at the tangential position R8 in Figure 2C corresponds to the outer diameter of the real product 4.8. The same thing holds for the outer diameter at other positions R.i (i= 7,9,10,11,12). The determined outer diameter for the virtual product 4' can also be an average of the six outer diameters B.i (i= 7,8,..12) of the virtual product 4'.

What has been set out above holds entirely analogously for the inner diameter of the virtual product. Thus, an inner diameter C.i can be determined of the virtual product from the distance A2.i and A2'.i associated with the sensors 10.i and 10'.i from Figure 2C. This inner diameter is E-A2.i-A2'.i. In this way, six inner diameters are determined which have respectively been obtained on the basis of the products 4.i (i= 7,8,..12). This means that the inner diameter of the virtual product 4' can vary in tangential direction. In this example, with the virtual product the position of the inner diameters C.7 and C.11, C.10 and C.9, C.8 and C.12 coincides. These pairs can be averaged for a better estimate of the inner diameter of the virtual product at the respective position. It is thus possible that the inner diameter is determined for the virtual product depending on a tangential position phi. This is because the inner diameter for different tangential positions on the inner surface of the virtual product corresponds to the inner diameter that has been determined in real products whose rotational position corresponds to a tangential position at the virtual product. Thus, the inner diameter at the tangential position R8 in Figure 2C corresponds to the inner diameter of the real product 4.8. The same thing holds for the inner diameter at other positions R.i (i= 7,9,10,11,12).

Preferably, it holds that inspecting also comprises a controlling, whereby at least one step of the steps a., b. and c. is adjusted on the basis of measuring results obtained with the aid of the at least one sensor (such as the distances A1, A2, A1', A2', the above-mentioned diameters and/or skew positions) and possibly the determined rotational positions, more particularly on the basis of the determined lateral glass thickness distribution of the virtual product. This controlling may then be determined and carried out by the signal processing unit 14 which to this end issues control signals via the line 30.

According to a practical embodiment, it holds that a plurality of the steps b. are carried out parallel to each other for producing parallel to each other a plurality of the products in a plurality of production flows which each comprise a step b., wherein each lateral glass thickness distribution of a virtual product has been obtained on the basis of measurements with the at least one sensor on products that have been manufactured in a same production flow.

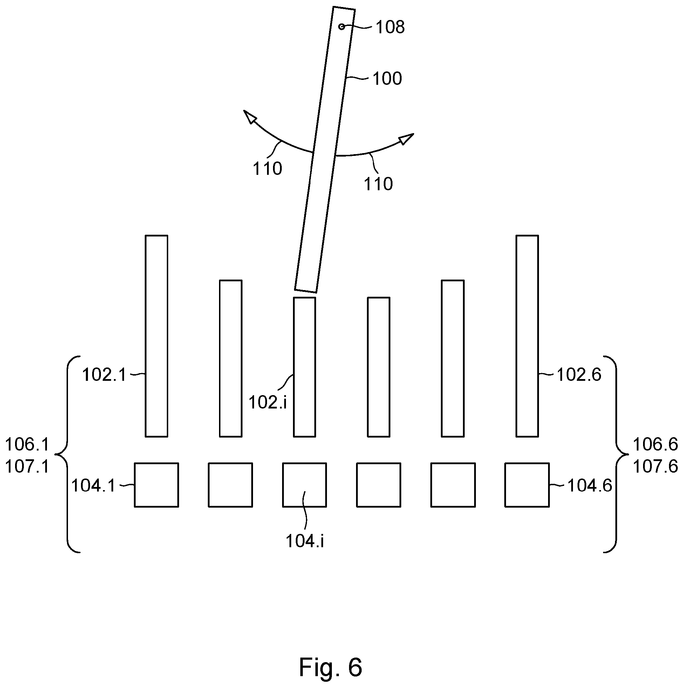

In such a practical embodiment it is for instance possible that six products are formed parallel to each other in six parallel production flows. To this end, the system, in this example the product forming apparatus 3, includes six production flow paths 106.j which, for instance, each comprise at least one trough and a mould. This is shown in Figure 6 in which a glass gob formed in the heating apparatus 2 is guided via a switch 100 to a production flow path 106.j (j=1,2,3,..6) selected with the switch, each production flow path 106.j comprising at least one trough 102.j and a mould 104.j. The glass gobs that are successively transported along the production flow path 106.j together form a production flow 107.j. By rotation of the switch in the direction of arrows 110 around an axis (pivot) 108, other production flow paths 106.j can be selected with the switch. The glass gob flows, in this example, via the selected at least one trough 102.j to the mould 104.j. In this example, for j, successively the value 1,2,3,4,5,6 is selected by the switch 100. In this example there are thus six parallel production flows.

Because there are six production flows, in succession six products 4.1, 4.2, 4.3, ...4.6 are formed which are placed in a row of six products on the conveyor. Here, product 4.1 has been formed from a glass gob which has been transported via production flow path 106.1 in production flow 107.1, product 4.2 has been formed from a glass gob which has been transported via production flow path 106.2 in production flow 107.2, product 4.3 has been formed from a glass gob which has been transported via production flow path 106.3 in production flow 107.3, etc. More generally, product 4.j has been formed from a glass gob which has been transported via production flow path 106.j in production flow 107.j, for j = 1,2,3,4,5,6. When thus six products have been produced, this process repeats itself.

Here, product 4.7 is formed from a glass gob which has been transported via production flow path 106.1 in production flow 107.1, product 4.8 is formed from a glass gob which has been transported via production flow path 106.2 in production flow 107.2, product 4.9 is formed from a glass gob which has been transported via production flow path 106.3 in production flow 107.3, etc. More generally, product 4.j+6 has been formed from a glass gob which has been transported via production flow path 106.j in production flow 107.j, for j = 1,2,3,4,5,6. When thus six products have been produced, this process repeats itself. Generally, therefore, it holds that product 4.j+n.6 with n=0,1,2,3,.... has been formed from a glass gob which has been transported via production flow path 106.j in production flow 107.j for j = 1,2,3,4,5,6.

The products 4.j, 4.j+6, 4.j+12, ....4.j+n.6 have then been made in a same production flow j, where j= 1,2,3,4,5,6 and n= 0,1,2,3,4,.....According to this variant, a number of successive measurements on products 4.j+n.6 for different values of n and a same value of j (and which thus belong to a same production flow j) are processed in combination with the signal processing unit for obtaining a glass thickness distribution of a virtual glass product. This number may for instance be eight. Thus, for a value of j, measurements on the products for n=0,1,2,..7 can be combined for obtaining a glass thickness distribution of a virtual product 4'. Next, for the same value of j, measurements on the products for n=8, 9, 10, ..15 can be processed in combination with the signal processing unit for obtaining a glass thickness distribution of another virtual product 4', etc. These measurements are obtained spread in time, and have been obtained from products that have been produced with the aid of the production flow path 106.j in the production flow 107.j. This makes it possible to detect changes in the glass thickness distribution of successive virtual products as a result of for instance wear in the at least one trough and/or mould of production flow path 106.j or changes in the glass thickness distribution of successive virtual products as a result of drifting settings in production flow path 106.j. When such a change exceeds a limiting value, automatically or manually. settings of production flow path 106.j can be changed (such as a position and/or orientation of a trough and/or mould of the respective production flow path), a trough of production flow path 106.j can be lubricated, a mould and/or trough of production flow path 106.j can be replaced, etc. It is thus possible, in case of a change in glass thickness distributions successively obtained spread in time and the change exceeding a predetermined value, to correct manually or automatically under control of the signal processing unit 14 via line 30. It is also possible, in case of a deviation in a single glass thickness distribution and the deviation exceeding a predetermined value, to correct manually or automatically under control of the signal processing unit via line 30. Correcting may for instance be understood to mean adjusting a position and/or orientation of a trough and/or mould, providing a trough with a lubricant and/or replacing a mould.

All this can also be carried out for all other possible values of j so that glass thickness distributions are obtained for different virtual glass products relating to different production flow paths 106.j and hence to different production flows 107.j. If there is a deviation in a glass thickness distribution of a virtual product 4' that has been obtained from a production flow j, for instance the position of a trough and a mould belonging to the production flow path j may be corrected relative to each other, or a trough belonging to the production flow path j may be provided with a lubricant. Also, a mould of the production flow path j. may be replaced. All of this may be carried out manually or automatically via line 30 under control of the signal processing unit. Also, changes in glass thickness distributions of virtual products that have successively been obtained with the aid of a same production flow path 106.j can be detected by the signal processing unit. If these changes exceed a predetermined value, again, as discussed above, settings of the respective production flow path 106.j may be adjusted, a trough of the respective production flow path 106.j may be lubricated and/or a mould of the respective production flow path may be replaced, manually or automatically.

In this example, in each case, eight measurements on products that have been obtained from a same production flow j were processed in combination for obtaining information about the glass thickness distribution of a virtual product. Instead of using in each case a= 8 measurements, the signal processing unit may also be configured to choose a to be variable, for instance such that a sufficient number of successive measurements are selected in order for these measurements in combination to cover the associated virtual product around the axial axis of the virtual product with a mutual distance in tangential direction between neighbouring measurements (or, to put it better, positions of measurements) that is smaller than a predetermined value (as discussed hereinbefore in the context of a single production flow according to Figure 1A).

The signal processing unit then decides automatically which and how many measurements in each case are processed in combination for computing a glass thickness distribution of a virtual product associated with a particular production flow path j. Accordingly, it holds in particular according to the invention that a plurality of the steps b. are carried out parallel to each other for producing parallel to each other a plurality of the products in a plurality of production flows which each comprise a step b., wherein each glass thickness distribution of a virtual product has been obtained on the basis of measurements of products that have been manufactured in a same production flow, more particularly wherein on the basis of at least one determined glass thickness distribution of a virtual product that has been obtained from measurements on products that stem from a same production flow, in an automatic manner the production flow is controlled, as by adjusting of settings of that production flow.

Further, it holds thus, in particular, according to the invention, that the system is configured such that, in use, a plurality of the steps b. are carried out parallel to each other for producing parallel to each other a plurality of the products in a plurality of production flows which each comprise a step b., wherein the signal processing unit is configured such that, in use, each glass thickness distribution of a virtual product is obtained on the basis of measurements on products that have been manufactured in a same production flow, more particularly wherein on the basis of at least one determined glass thickness distribution of at least one virtual product that has been obtained from measurements on products that stem from a same production flow, in an automatic manner the production flow is controlled for instance in that settings of that production flow are adjusted.

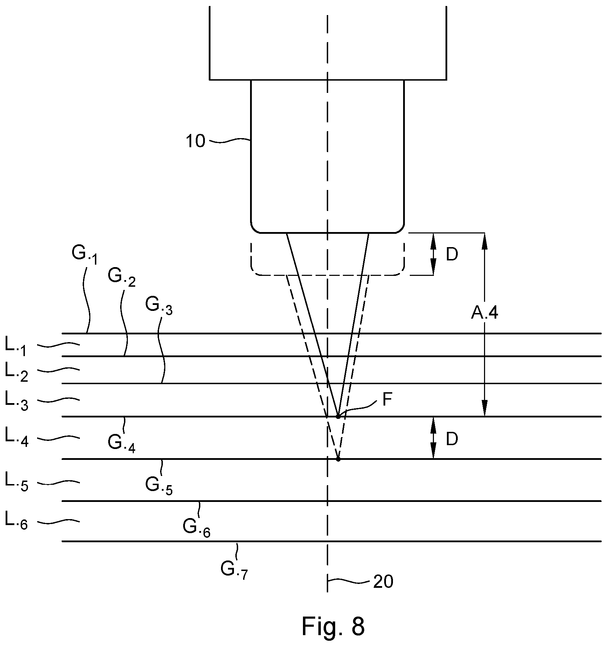

Instead of a chromatic confocal sensor as described hereinbefore, other sensors may be used to measure a glass thickness in the systems and methods defined hereinbefore. For instance, the sensor according to Figure 7 may be adapted in order that it emits only one colour of light and so has only one focus (see Figure 8). By moving the sensor of Figure 8 back and forth in its measuring direction 20, the sensor can detect when its focus F coincides with one of the boundary surfaces G.g (g = 1,2,3,...7) because the light is then strongly reflected and detected by the sensor. In Figure 8 it has been chosen, as an example, for the focus F to coincide with the boundary surface G.4. Thereupon, the sensor is moved in the direction of the boundary surface G.5 and it can be detected with the sensor when the focus F coincides with the boundary surface G.5. Then too, the intensity of the received light will have a peak. This situation is indicated in Figure 8 with dotted lines. To this end, the sensor has been moved over a distance D. This distance then corresponds to the thickness D of the layer L.4. By repeating this for all boundary surfaces, the thickness of each layer can be determined from the measured differences in positions of the sensor. It will be clear, for that matter, that for a product 4.i the wall thickness at a particular position of the product can be determined from the difference in positions of the sensor where a peak in reflections on the inner wall and the outer wall of the product, respectively, is received. In the embodiments outlined hereinbefore, sensor 10 of Figure 1A should then be set up in a manner movable in its measuring direction. It will also be clear that in this manner a skew position of a product can be measured by comparing the position of the sensor where reflection on the outer surface of the product occurs with the position of the same or a different sensor at a different height where reflection on the outer surface occurs. The difference in position measured in the measuring direction then corresponds to a skew position, if present. Also when the sensors 10 and 10' as discussed with reference to Figure 4 are replaced with sensors that radiate light of a single frequency and which are set up in a manner movable in their viewing direction, an inner and outer diameter of the product can be determined from the positions of the sensors where reflection occurs on the inner wall and the outer wall, respectively, from the associated positions of the sensors. Other sensors that may be used for performing a thickness measurement are for instance laser interference sensors.

Finally, it is noted that automatic control loops via line 30 can comprise the adjusting (adapting), on the basis of a determined glass thickness distribution, of:
- Feeder temperature and temperature distribution
- Gob temperature distribution
- Gob forming process
- Gob loading process
- Mould cooling and residence time in the mould
- Design of the parison and the preform moulds
- Blowout process (B&B process)
- Plunger press process (NNPB, PB process)
- Plunger cooling process
- Blowout process at the front
- Shape and design of the blowing pipe
- Optimal lubricating method and lubricant moulds
- Optimal standing time determination of the moulds
- Optimal timing setting of the I.S. machine (glass forming machine)
- Optimal temperature settings of moulds and automatic control thereof
- Optimal air pressure control of product blowout
- Optimal air pressure control for making the parison
- Active reheating for an optimal temperature distribution of the glass gobs for an optimal LGD

Such variants each fall within the scope of the invention.

## Claims

1. Method for inspecting hollow glass products (4) of glass product material, wherein the glass products are manufactured by:
a. heating the glass product material;
b. forming the heated glass product material into at least one glass product in a production flow (107);
c. cooling the formed glass product;
**characterised in that** inspecting the glass products comprises the following steps:
d. transporting the glass products formed in step b. successively along a predetermined path (106) along at least one sensor (10), such as a Chromatic Confocal sensor, for measuring a glass thickness, wherein, using the at least one sensor, of a plurality of the glass products that are transported successively along the at least one sensor, per glass product at least one glass thickness is measured, wherein step d. is carried out between steps b. and c. and wherein in step d. of each glass product of the plurality of glass products the rotational position (R) of the glass product around an axial axis (A) of the glass product relative to the at least one sensor is determined, wherein in a step e. the determined glass thicknesses and the associated rotational positions of the plurality of products are processed in combination for obtaining information about a lateral glass thickness distribution around an axial axis of a virtual glass product (4') that represents an average of the plurality of glass products.

2. Method according to claim 1, **characterised in that** in step d. the product is transported in a horizontally directed (b) plane, wherein the axial axis of the product is vertically directed (h).

3. Method according to claim 1 or 2, **characterised in that** the glass thickness distribution indicates relative variations in glass thickness, and/or **characterised in that** the glass thickness distribution comprises absolute values of the glass thickness distribution.

4. Method according to any one of the preceding claims, **characterised in that** on a sufficiently large number of products from a production flow measurements with the at least one sensor are performed in order that these measurements in combination cover the associated virtual product around the axial axis of the virtual product such that each neighbouring distance between the positions of the measurements at the virtual product in tangential direction (T) is smaller than a predetermined value.

5. Method according to any one of the preceding claims, **characterised in that** in step e. the lateral glass thickness distribution is determined in an area (26.1) of the virtual glass product that extends around the axial axis of the virtual glass product.

6. Method according to according to any one of the preceding claims, **characterised in that** step e. is carried out repeatedly for obtaining a lateral glass thickness distribution in a second area (26.2) of the virtual glass product that extends around the axial axis of the virtual glass product, wherein the first and second area are staggered with respect to each other in the axial direction, wherein optionally step e. is respectively carried out repeatedly at least three times for respectively obtaining lateral glass thickness distributions in respectively at least three mutually different areas which each extend around the axial axis of the virtual glass product and are staggered with respect to each other in axial direction and which preferably in combination cover at least substantially the whole virtual glass product, and/or wherein optionally in step d. the sensor is displaced in the axial direction for obtaining recordings in the different areas.

7. Method according to any one of the preceding claims, **characterised in that** a plurality of the steps b. are carried out parallel to each other for producing parallel to each other a plurality of the products in a plurality of production flows (107) each comprising a step b., wherein each lateral glass thickness distribution of a virtual product has been obtained on the basis of measurements with the at least one sensor on products that have been manufactured in a same production flow.

8. Method according to any one of the preceding claims, **characterised in that** in step d. with the at least one sensor successively wall thicknesses are determined of a glass product that is transported along the at least one sensor so that the determined glass thicknesses relate to different positions of a wall of the glass product, these positions being separate from each other in a direction (8) in which the product is transported in step d; and/or
**in that** in step d. the product is transported in a horizontal plane, wherein the at least one sensor is moved in vertical direction for measuring on the glass product at different heights (D), optionally **characterised in that** at different heights a glass thickness of the product is determined, and/or optionally **characterised in that** from the measuring results obtained with the at least one sensor a skew position of the product is determined.

9. Method according to any one of the preceding claims, **characterised in that** with the at least one sensor in each case a glass thickness is determined of a part (Q) of a wall of a glass product of which an outer surface faces the at least one sensor, optionally **characterised in that** with the at least one sensor a first distance (A1) is measured between the at least one sensor and the outer surface (O) of the part of the wall and a second distance (A2) is measured between an inner surface (I) of the part of the wall, wherein from the first distance and the second distance the glass thickness of the part of the wall is determined.

10. Method according to any one of the preceding claims, **characterised in that** in step d. use is made of at least two of the sensors (10,10'), wherein the at least two sensors are opposite to each other and face each other and wherein the products are transported between the at least two sensors, wherein optionally with a first sensor (10) of the at least two sensors a first glass thickness of a first part of the wall of the glass product is determined and that with a second sensor (10') of the at least two sensors a second glass thickness of a second part of the wall of the glass product is determined, wherein the first part and the second part are on sides of the glass product that are located opposite to each other, and/or wherin optionally with the first sensor a first distance (A1) is measured between the first sensor and an outer surface (O) of a first part of the wall that faces the first sensor and that with the second sensor a second distance (A1') is measured between the second sensor and an outer surface (O) of a second part of the wall that faces the second sensor, wherein from the first and second distance an outer diameter of the glass product is determined, and/or wherein optionally with the first sensor a third distance (A2) is measured between the first sensor and an inner surface (I) of a first part of the wall that faces the first sensor and that with the second sensor a fourth distance (A2') is measured between the second sensor and an inner surface (I) of a second part of the wall that faces the second sensor, wherein from the third and fourth distance an inner diameter of the glass product is determined, and/or wherein optionally per product with the first sensor a first distance is measured between the first sensor and an outer surface of a first part of the wall that faces the first sensor and that with the second sensor a second distance is measured between the second sensor and an outer surface of a second part of the wall that faces the second sensor, wherein from the first and second distance determined for different products and associated rotational position an outer diameter (B) and/or skew position of the virtual product is determined.

11. Method according to any one of the preceding claims, **characterised in that** the glass products between steps b. and c. are transported on a conveyor (6) along the path, wherein in particular each product between steps b. and c. is placed on the conveyor with a rotational position around an axial axis of the glass product that varies per glass product, wherein optionally the glass product between steps b. and c. is transported on the conveyor from a product forming apparatus (3) such as a mould (104) in which the glass product has been formed in step b. to a cooling apparatus (7) in which the product is cooled in step c, wherein optionally the rotational position of each of the glass products of the plurality of glass products around its axial axis on the conveyor is determined.

12. Method according to any one of the preceding claims, **characterised in that** with the aid of at least one rotational position measuring unit (11) such as a camera the rotational position of a glass product of the plurality of glass products is determined, wherein optionally with the rotational position measuring unit an image of the glass product is made, wherein in the image a predetermined marking (M) on the glass product such as a seam and/or a dot is detected for determining the rotational position.

13. Method according to any one of the preceding claims, **characterised in that** inspecting also comprises a controlling whereby at least one step of the steps a., b. and c. is adjusted on the basis of measuring results obtained with the aid of the at least one sensor and possibly the determined rotational positions, more particularly on the basis of the determined lateral glass thickness distribution of the virtual product.

14. Method for producing and inspecting hollow glass products (4) of glass product material, wherein the glass products are at least manufactured by:
a. heating the glass product material;
b. forming the heated glass product material into a glass product in a production flow;
c. cooling the formed glass product; **characterised in that** inspecting the glass products is performed according to any of claims 1-13.

15. System (1) for inspecting glass products (4) of glass product material according to the method according to the pre-characterising portion of claim 1, wherein the system comprises:
at least one sensor (10) for scanning the glass products; and
a signal processing unit (14) connected with the at least one sensor for processing signals coming from the at least one sensor, **characterised in that** the at least one sensor is a sensor, such as a Chromatic Confocal sensor, for measuring a glass thickness, wherein the system further comprises a rotational position unit (11) such as a camera for obtaining information which represents a rotational position (R) relative to the at least one sensor and around an axial axis (A) of a glass product on which measuring has been done with the at least one sensor, wherein, in use, the at least one sensor is set up such that the glass products are successively measured with the at least one sensor, wherein the signal processing unit is configured for, per scanned product, on the basis of measuring results of the at least one sensor and the associated information of the rotational position unit, determining a lateral glass thickness distribution around an axial axis of a virtual glass product (4') that represents the plurality of glass products so that the system is further configured for carrying out the method according to the characterising portion of claim 1.

16. System (1) for producing and inspecting glass products (4) of glass product material according to the method of the pre-characterising portion of claim 14, comprising the system of claim 15, wherein the system further comprises:
a heating apparatus (2) for carrying out step a.;
a product forming apparatus (3) such as a mould (104) for carrying out step b.;
a cooling apparatus (7) for carrying out step c.; and
a transporting apparatus (6) for transporting glass products formed with the product forming apparatus from the product forming apparatus to the cooling apparatus.

17. System according to claim 15 or 16, **characterised in that** the system is further configured for carrying out the method according to any one of the claims 2-13.

18. System according to one of the preceding claims 15, 16 or 17, **characterised in that** the system is configured such that, in use, a plurality of the steps b. are carried out parallel to each other for producing parallel to each other a plurality of the glass products in a plurality of production flows (107) which each comprise a step b., wherein the signal processing unit (14) is configured such that, in use, the determined glass thicknesses and the associated rotational positions of the plurality of glass products coming from a same production flow are processed in combination for obtaining information about a lateral glass thickness distribution around an axial axis of a virtual glass product (4') that represents the plurality of glass products from a same production flow.

## Patentansprüche

1. Verfahren zur Inspektion von Hohlglasprodukten (4) aus Glasproduktmaterial, wobei die Glasprodukte hergestellt werden durch:
a. Erhitzen des Glasproduktmaterials;
b. Formen des erhitzten Glasproduktmaterials in mindestens ein Glasprodukt in einem Produktionsfluss (107);
c. Abkühlen des geformten Glasprodukts;
**dadurch gekennzeichnet, dass** die Inspektion des Glasprodukts die folgenden Schritte umfasst:
d. Transportieren der in Schritt b geformten Glasprodukte nacheinander entlang eines vorbestimmten Pfads (106) entlang mindestens eines Sensors (10), z. B. ein chromatisch-konfokaler Sensor zur Messung einer Glasdicke, wobei, mit dem mindestens einen Sensor, einer Vielzahl der Glasprodukte, die nacheinander entlang des mindestens einen Sensors transportiert werden, pro Glasprodukt mindestens eine Glasdicke gemessen wird, wobei Schritt d zwischen den Schritten b und c ausgeführt wird und wobei in Schritt d jedes Glasprodukts der Vielzahl von Glasprodukten die Drehposition (R) des Glasprodukts um eine axiale Achse (A) des Glasprodukts relativ zu dem mindestens einen Sensor bestimmt wird, wobei in einem Schritt e die bestimmten Glasdicken und die zugehörigen Drehpositionen der Vielzahl von Produkten in Kombination verarbeitet werden, um Informationen über eine laterale Verteilung der Glasdicke um eine axiale Achse eines virtuellen Glasprodukts (4') zu erhalten, das einen Durchschnitt der Vielzahl der Glasprodukte repräsentiert.

2. Verfahren nach Anspruch 1, **dadurch gekennzeichnet, dass** in Schritt d das Produkt in einer horizontal ausgerichteten (b) Ebene transportiert wird, wobei die axiale Achse des Produkts vertikal ausgerichtet (h) ist.

3. Verfahren nach Anspruch 1 oder 2, **dadurch gekennzeichnet, dass** die Verteilung der Glasdicke relative Schwankungen der Glasdicke angibt und/oder **dadurch gekennzeichnet, dass** die Verteilung der Glasdicke absolute Werte der Verteilung der Glasdicke umfasst.

4. Verfahren nach einem der vorhergehenden Ansprüche, **dadurch gekennzeichnet, dass** Messungen mit dem mindestens einen Sensor an einer ausreichend großen Anzahl an Produkten aus einem Produktionsfluss durchgeführt werden, damit diese Messungen in Kombination das zugehörige virtuelle Produkt um die axiale Achse des virtuellen Produkts abdecken, so dass jeder benachbarte Abstand zwischen den Positionen der Messungen an dem virtuellen Produkt in tangentialer Richtung (T) kleiner ist als ein vorbestimmter Wert.

5. Verfahren nach einem der vorhergehenden Ansprüche, **dadurch gekennzeichnet, dass** in Schritt e die laterale Verteilung der Glasdicke in einem Bereich (26.1) des virtuellen Glasprodukts, das sich um die axiale Achse des virtuellen Glasprodukts erstreckt, bestimmt wird.

6. Verfahren nach einem der vorhergehenden Ansprüche, **dadurch gekennzeichnet, dass** Schritt e wiederholt ausgeführt wird, um eine laterale Verteilung der Glasdicke in einem zweiten Bereich (26.2) des virtuellen Glasprodukts zu erhalten, der sich um die axiale Achse des virtuellen Glasprodukts erstreckt, wobei der erste und zweite Bereich in axialer Richtung zueinander gestaffelt sind, wobei optional der Schritt e jeweils mindestens dreimal wiederholt wird, um laterale Verteilungen der Glasdicke in mindestens drei voneinander unterschiedlichen Bereichen zu erhalten, die sich jeweils um die axiale Achse des virtuellen Glasprodukts erstrecken und zueinander in axialer Richtung gestaffelt sind und die vorzugsweise in Kombination mindestens im Wesentlichen das gesamte virtuelle Glasprodukt abdecken und/oder wobei optional in Schritt d der Sensor in axialer Richtung verschoben wird, um Aufzeichnungen in verschiedenen Bereichen zu erhalten.

7. Verfahren nach einem der vorhergehenden Ansprüche, **dadurch gekennzeichnet, dass** eine Vielzahl der Schritte b parallel zueinander ausgeführt wird, um eine Vielzahl der Produkte in einer Vielzahl von Produktionsflüssen (107), die jeweils einen Schritt b umfassen, parallel zueinander herzustellen, wobei jede laterale Verteilung der Glasdicke eines virtuellen Produkts auf Basis der Messungen mit dem mindestens einen Sensor an Produkten, die in demselben Produktionsfluss hergestellt wurden, zu erhalten.

8. Verfahren nach einem der vorhergehenden Ansprüche, **dadurch gekennzeichnet, dass** in Schritt d. mit dem mindestens einen Sensor nacheinander Wanddicken eines Glasprodukts, das entlang des mindestens einen Sensors transportiert wird, bestimmt werden, so dass die bestimmten Glasdicken sich auf verschiedene Positionen einer Wand des Glasprodukts beziehen, wobei diese Positionen getrennt voneinander sind in einer Richtung (8), in der das Produkt in Schritt d transportiert wird; und/oder
dass in Schritt d das Produkt in einer horizontalen Ebene transportiert wird, wobei der mindestens eine Sensor in vertikale Richtung bewegt wird, um das Glasprodukt an verschiedenen Höhen (D) zu messen, optional **dadurch gekennzeichnet, dass** eine Glasdicke des Produkts an verschiedenen Höhen bestimmt wird und/oder optional **dadurch gekennzeichnet, dass** eine Schräglage des Produkts aus den mit dem mindestens einen Sensor erhaltenen Messungen bestimmt wird.

9. Verfahren nach einem der vorhergehenden Ansprüche, **dadurch gekennzeichnet, dass** mit dem mindestens einen Sensor jeweils eine Glasdicke eines Teils (Q) einer Wand eines Glasprodukts, bei dem eine Außenfläche dem mindestens einen Sensor zugewandt ist, bestimmt wird, optional **dadurch gekennzeichnet, dass** mit dem mindestens einen Sensor ein erster Abstand (A1) zwischen dem mindestens einen Sensor und der Außenfläche (O) des Teils der Wand gemessen wird und ein zweiter Abstand (A2) zwischen einer Innenfläche (I) des Teils der Wand gemessen wird, wobei die Glasdicke des Teils der Wand aus dem ersten Abstand und dem zweiten Abstand bestimmt wird.

10. Verfahren nach einem der vorhergehenden Ansprüche, **dadurch gekennzeichnet, dass** in Schritt d mindestens zwei der Sensoren (10, 10') verwendet werden, wobei die mindestens zwei Sensoren sich gegenüberliegend voneinander befinden und einander zugewandt sind und wobei die Produkte zwischen den mindestens zwei Sensoren transportiert werden, wobei eine erste Glasdicke eines ersten Teils der Wand des Glasprodukts optional mit einem ersten Sensor (10) der mindestens zwei Sensoren bestimmt wird und dass eine zweite Glasdicke eines zweiten Teils der Wand des Glasprodukts mit einem zweiten Sensor (10') der mindestens zwei Sensoren bestimmt wird, wobei sich der erste Teil und der zweite Teil an Seiten des Glasprodukts befinden, die gegenüberliegend voneinander sind und/oder wobei ein erster Abstand (A1) zwischen dem ersten Sensor und einer Außenfläche (O) einer ersten Teils der Wand, die dem ersten Sensor zugewandt ist, optional mit dem ersten Sensor gemessen wird und dass ein zweiter Abstand (A1') zwischen dem zweiten Sensor und einer Außenfläche (O) eines zweiten Teils der Wand, die dem zweiten Sensor zugewandt ist, gemessen wird, wobei aus dem ersten und dem zweiten Abstand ein Außendurchmesser des Glasprodukts bestimmt wird und/oder wobei optional mit dem ersten Sensor ein dritter Abstand (A2) zwischen dem ersten Sensor und einer Innenfläche (I) eines ersten Teils der Wand, die dem ersten Sensor zugewandt ist, gemessen wird und dass mit dem zweiten Sensor ein vierter Abstand (A2) zwischen dem zweiten Sensor und einer Innenfläche (I) eines zweiten Teils der Wand, der dem zweiten Sensor zugewandt ist, gemessen wird, wobei aus dem dritten und dem vierten Abstand ein Innendurchmesser des Glasprodukts bestimmt wird, und/oder wobei optional pro Produkt ein erster Abstand mit dem erste Sensor gemessen wird zwischen dem ersten Sensor und einer Außenfläche eines ersten Teils der Wand, die dem ersten Sensor zugewandt ist und dass mit dem zweiten Sensor ein zweiter Abstand zwischen dem zweiten Sensor und einer Außenfläche eines zweiten Teils der Wand, die dem zweiten Sensor zugewandt ist, gemessen wird, wobei aus dem ersten und dem zweiten Abstand ein Außendurchmesser (B) und/oder eine Schräglage des virtuellen Produkts für verschiedene Produkte und zugehörige Drehpositionen bestimmt wird.

11. Verfahren nach einem der vorhergehenden Ansprüche, **dadurch gekennzeichnet, dass** die Glasprodukte zwischen den Schritten b und c auf einem Förderband (6) entlang des Pfads transportiert werden, wobei insbesondere jedes Produkt zwischen den Schritten b und c auf dem Förderband mit einer Drehposition um eine axiale Achse des Glasprodukts, das je nach Glasprodukt variiert, platziert wird, wobei das Glasprodukt zwischen den Schritten b und c optional auf dem Förderband transportiert wird von einem Produktformgerät (3), z. B. einer Gießform (104), in dem das Glasprodukt in Schritt b geformt wurde zu einem Kühlgerät (7), in dem das Produkt in Schritt c abgekühlt wird, wobei optional die Drehposition jedes Glasprodukts der Vielzahl von Glasprodukten um seine axiale Achse auf dem Förderband bestimmt wird.

12. Verfahren nach einem der vorhergehenden Ansprüche, **dadurch gekennzeichnet, dass** mit Hilfe mindestens eines Drehpositionsmessgeräts (11), z. B. einer Kamera, die Drehposition eines Glasprodukts der Vielzahl von Glasprodukten bestimmt wird, wobei optional mit dem Drehpositionsmessgerät ein Bild des Glasprodukts gemacht wird, wobei auf dem Bild eine vorbestimmte Markierung (M) auf dem Glasprodukt, z. B. eine Fuge und/oder ein Punkt erkannt wird, um die Drehposition zu bestimmen.

13. Verfahren nach einem der vorhergehenden Ansprüche, **dadurch gekennzeichnet, dass** die Inspektion auch eine Kontrolle umfasst, wobei mindestens ein Schritt der Schritte a, b und c auf Basis der Messergebnisse eingestellt wird, die mit Hilfe des mindestens einen Sensors und möglicherweise den ermittelten Drehpositionen erzielt wurden, insbesondere auf Basis der ermittelten lateralen Verteilung der Glasdicke des virtuellen Produkts.

14. Verfahren zur Herstellung und Inspektion von Hohlglasprodukten (4) aus Glasproduktmaterial, wobei die Glasprodukte mindestens hergestellt werden durch:
a. Erhitzen des Glasproduktmaterials;
b. Formen des erhitzten Glasproduktmaterials in ein Glasprodukt in einem Produktionsfluss;
c. Abkühlen des geformten Glasprodukts; **dadurch gekennzeichnet, dass** die Inspektion des Glasprodukts nach einem der Ansprüche 1 bis 13 erfolgt.

15. System (1) zur Inspektion von Glasprodukten (4) aus Glasproduktmaterial nach dem Verfahren gemäß dem Oberbegriff von Anspruch 1, wobei das System umfasst:
mindestens einen Sensor (10) zum Scannen der Glasprodukte; und
eine Signalverarbeitungseinheit (14), die mit dem mindestens einen Sensor verbunden ist, um Signale, die von dem mindestens einen Sensor kommen, zu verarbeiten, **dadurch gekennzeichnet, dass** der mindestens eine Sensor ein Sensor, z. B. ein chromatisch-konfokaler Sensor, zur Messung einer Glasdicke ist, wobei das System weiter eine Drehpositionseinheit (11) umfasst, z. B. eine Kamera, um Informationen zu erhalten, die eine Drehposition (R) relativ zu dem mindestens einen Sensor und um eine axiale Achse (A) eines Glasprodukts darstellt, an dem die Messung mit dem mindestens einen Sensor durchgeführt wurde, wobei, in Benutzung, der mindestens eine Sensor so eingerichtet ist, dass die Glasprodukte nacheinander mit dem mindestens einen Sensor gemessen werden, wobei die Signalverarbeitungseinheit dazu eingerichtet ist, pro gescanntem Produkt auf Basis der Messergebnisse des mindestens einen Sensors und den zugehörigen Informationen der Drehpositionseinheit, eine laterale Verteilung der Glasdicke um eine axiale Achse eines virtuellen Glasprodukts (4') zu bestimmen, das die Vielzahl von Glasprodukten repräsentiert, so dass das System weiter dazu eingerichtet ist, das Verfahren nach dem kennzeichnenden Teil von Anspruch 1 auszuführen.

16. System (1) zur Herstellung und Inspektion von Glasprodukten (4) aus Glasproduktmaterial nach dem Verfahren des Oberbegriffs von Anspruch 14, aufweisend das System nach Anspruch 15, wobei das System weiter aufweist:
ein Heizgerät (2) zum Ausführen von Schritt a;
ein Produktformgerät (3), z. B. eine Gießform (104) zum Ausführen von Schritt b;
ein Kühlgerät (7) zum Ausführen von Schritt c; und
ein Transportgerät (6) zum Transportieren des Glasprodukts, das mit dem Produktformgerät geformt wurde, vom Produktformgerät zum Kühlgerät.

17. System nach Anspruch 15 oder 16, **dadurch gekennzeichnet, dass** das System weiter dazu eingerichtet ist, das Verfahren nach einem der Ansprüche 2 bis 13 auszuführen.

18. System nach einem der vorhergehenden Ansprüche 15, 16 oder 17, **dadurch gekennzeichnet, dass** das System so eingerichtet ist, dass, in Benutzung, eine Vielzahl der Schritte b parallel zueinander ausgeführt wird, um parallel zueinander eine Vielzahl der Glasprodukte in einer Vielzahl von Produktionsflüssen (107) herzustellen, welche jeweils einen Schritt b aufweisen, wobei die Signalverarbeitungseinheit (14) so konfiguriert ist, dass, in Benutzung, die ermittelte Glasdicke und die zugehörigen Drehpositionen der Vielzahl von Glasprodukten, die aus demselben Produktionsfluss kommen, in Kombination verarbeitet werden, um Informationen über eine laterale Verteilung der Glasdicke um eine axiale Achse eines virtuellen Glasprodukts (4') zu erhalten, das die Vielzahl der Glasprodukte aus demselben Produktionsfluss repräsentiert.

## Revendications

1. Procédé d'inspection de produits en verre creux (4) en matériau de produit en verre, dans lequel les produits en verre sont fabriqués en :
a. chauffant le matériau de produit en verre ;
b. formant le matériau de produit en verre chauffé en au moins un produit en verre dans un flux de production (107) ;
c. refroidissant le produit en verre formé ;
**caractérisé en ce que** l'inspection des produits en verre comprend les étapes suivantes :
d. le transport des produits en verre formés à l'étape b. successivement le long d'un trajet prédéterminé (106) le long d'au moins un capteur (10), tel qu'un capteur confocal chromatique, afin de mesurer une épaisseur du verre, dans lequel, à l'aide de l'au moins un capteur, au moins une épaisseur de verre d'une pluralité des produits en verre qui sont transportés successivement le long de l'au moins un capteur, pour chaque produit en verre, est mesurée, dans lequel l'étape d. est exécutée entre les étapes b. et c., et dans lequel, à l'étape d., pour chaque produit en verre de la pluralité de produits en verre, la position de rotation (R) du produit en verre autour d'un axe axial (A) du produit en verre par rapport à l'au moins un capteur est déterminée, dans lequel, à une étape e., l'épaisseur de verre déterminée et les positions de rotation associées de la pluralité de produits sont traitées en combinaison afin d'obtenir des informations sur une répartition d'épaisseur de verre latérale autour d'un axe axial d'un produit en verre virtuel (4') qui représente une moyenne de la pluralité de produits en verre.

2. Procédé selon la revendication 1, **caractérisé en ce que**, à l'étape d., le produit est transporté sur un plan orienté à l'horizontale (b), dans lequel l'axe axial du produit est orienté à la verticale (h).

3. Procédé selon la revendication 1 ou 2, **caractérisé en ce que** la répartition d'épaisseur de verre indique les variations relatives d'épaisseur du verre, et/ou **caractérisé en ce que** la répartition d'épaisseur de verre comprend des valeurs absolues de la répartition d'épaisseur de verre.

4. Procédé selon l'une quelconque des revendications précédentes, **caractérisé en ce que**, sur un nombre suffisamment élevé de produits issus d'un flux de production, des mesures avec l'au moins un capteur sont effectuées de sorte que ces mesures, en combinaison, couvrent le produit virtuel associé autour de l'axe axial du produit virtuel de sorte que chaque distance de voisinage entre les positions des mesures au niveau du produit virtuel dans la direction tangentielle (T) soit inférieure à une valeur prédéterminée.

5. Procédé selon l'une quelconque des revendications précédentes, **caractérisé en ce que**, à l'étape e., la répartition d'épaisseur de verre latérale est déterminée dans une zone (26.1) du produit en verre virtuel qui s'étend autour de l'axe axial du produit en verre virtuel.

6. Procédé selon l'une quelconque des revendications précédentes, **caractérisé en ce que** l'étape e. est exécutée de manière répétée afin d'obtenir une répartition d'épaisseur de verre latérale dans une deuxième zone (26.2) du produit en verre virtuel qui s'étend autour de l'axe axial du produit en verre virtuel, dans lequel la première et la deuxième zones sont décalées l'une par rapport à l'autre dans la direction axiale, dans lequel, éventuellement, l'étape e. est respectivement exécutée de manière répétée au moins trois fois afin d'obtenir respectivement des répartitions d'épaisseur de verre latérale dans respectivement au moins trois zones mutuellement différentes qui s'étendent chacune autour de l'axe axial du produit en verre virtuel et sont décalées les unes par rapport aux autres dans la direction axiale et qui, de préférence, en combinaison, couvrent au moins sensiblement le produit en verre virtuel dans son intégralité, et/ou dans lequel, éventuellement, à l'étape d., le capteur est déplacé dans la direction axiale afin d'obtenir des enregistrements dans les différentes zones.

7. Procédé selon l'une quelconque des revendications précédentes, **caractérisé en ce que** plusieurs des étapes b. sont exécutées parallèlement les unes aux autres afin de produire, parallèlement les unes aux autres, une pluralité des produits dans une pluralité de flux de production (107) comprenant chacun une étape b., dans lequel chaque répartition d'épaisseur de verre latérale d'un produit virtuel a été obtenue sur la base de mesures avec l'au moins un capteur sur des produits qui ont été fabriqués dans un même flux de production.

8. Procédé selon l'une quelconque des revendications précédentes, **caractérisé en ce que**, à l'étape d., avec l'au moins un capteur, de manière successive, les épaisseurs de parois d'un produit en verre qui est transporté le long de l'au moins un capteur sont déterminées de sorte que les épaisseurs de verre déterminées concernent différentes positions d'une paroi du produit en verre, ces positions étant distinctes les unes des autres dans une direction (8) dans laquelle le produit est transporté à l'étape d. ; et/ou
**en ce que**, à l'étape d., le produit est transporté sur un plan horizontal, dans lequel l'au moins un capteur est déplacé dans la direction verticale afin d'effectuer des mesures sur le produit en verre à différentes hauteurs (D), éventuellement **caractérisé en ce que**, à différentes hauteurs, une épaisseur de verre du produit est déterminée, et/ou, éventuellement, **caractérisé en ce que**, à partir des résultats des mesures obtenus avec l'au moins un capteur, une position d'inclinaison du produit est déterminée.

9. Procédé selon l'une quelconque des revendications précédentes, **caractérisé en ce que**, avec l'au moins un capteur, dans chaque cas, une épaisseur de verre est déterminée pour une partie (Q) d'une paroi d'un produit en verre dont une surface externe est tournée vers l'au moins un capteur, éventuellement **caractérisé en ce que**, avec l'au moins un capteur, une première distance (A1) est mesurée entre l'au moins un capteur et la surface externe (O) de la partie de la paroi et une deuxième distance (A2) est mesurée depuis une surface interne (I) de la partie de la paroi, dans lequel, à partir de la première distance et de la deuxième distance, l'épaisseur de verre de la partie de la paroi est déterminée.

10. Procédé selon l'une quelconque des revendications précédentes, **caractérisé en ce que**, à l'étape d., au moins deux des capteurs (10, 10') sont utilisés, dans lequel les au moins deux capteurs sont opposés l'un à l'autre et se font face, et dans lequel les produits sont transportés entre les au moins deux capteurs, dans lequel, éventuellement, avec un premier capteur (10) des au moins deux capteurs, une première épaisseur de verre d'une première partie de la paroi du produit en verre est déterminée, et **en ce que**, avec un deuxième capteur (10') des au moins deux capteurs, une deuxième épaisseur de verre d'une deuxième partie de la paroi du produit en verre est déterminée, dans lequel la première partie et la deuxième partie se trouvent sur les côtés du produit en verre qui sont opposés l'un à l'autre, et/ou dans lequel, éventuellement, avec le premier capteur, une première distance (A1) est mesurée entre le premier capteur et une surface externe (O) d'une première partie de la paroi qui fait face au premier capteur, et **en ce que**, avec le deuxième capteur, une deuxième distance (A1') est mesurée entre le deuxième capteur et une surface externe (O) d'une deuxième partie de la paroi qui fait face au deuxième capteur, dans lequel, à partir de la première et de la deuxième distances, un diamètre externe du produit en verre est déterminé, et/ou dans lequel, éventuellement, avec le premier capteur, une troisième distance (A2) est mesurée entre le premier capteur et une surface interne (I) d'une première partie de la paroi qui fait face au premier capteur, et **en ce que**, avec le deuxième capteur, une quatrième distance (A2') est mesurée entre le deuxième capteur et une surface interne (I) d'une deuxième partie de la paroi qui fait face au deuxième capteur, dans lequel, à partir de la troisième et de la quatrième distances, un diamètre interne du produit en verre est déterminé, et/ou dans lequel, éventuellement, pour chaque produit, avec le premier capteur, une première distance est mesurée entre le premier capteur et une surface externe d'une première partie de la paroi qui fait face au premier capteur, et **en ce que**, avec le deuxième capteur, une deuxième distance est mesurée entre le deuxième capteur et une surface externe d'une deuxième partie de la paroi qui fait face au deuxième capteur, dans lequel, à partir de la première et de la deuxième distances déterminées pour différents produits et de la position de rotation associée, un diamètre externe (B) et/ou une position d'inclinaison du produit virtuel est déterminé(e).

11. Procédé selon l'une quelconque des revendications précédentes, **caractérisé en ce que** les produits en verre entre les étapes b. et c. sont transportés sur un convoyeur (6) le long du trajet, dans lequel, en particulier, chaque produit entre les étapes b. et c. est placé sur le convoyeur avec une position de rotation autour d'un axe axial du produit en verre qui varie pour chaque produit en verre, dans lequel, éventuellement, le produit en verre entre les étapes b. et c. est transporté sur le convoyeur depuis un appareil de formation de produits (3) tel qu'un moule (104) dans lequel le produit en verre a été formé à l'étape b. vers un appareil de refroidissement (7) dans lequel le produit est refroidi à l'étape c., dans lequel, éventuellement, la position de rotation de chacun des produits en verre de la pluralité de produits en verre autour de son axe axial sur le convoyeur est déterminée.

12. Procédé selon l'une quelconque des revendications précédentes, **caractérisé en ce que**, avec l'aide d'au moins une unité de mesure de position de rotation (11) telle qu'une caméra, la position de rotation d'un produit en verre de la pluralité de produits en verre est déterminée, dans lequel, éventuellement, avec l'unité de mesure de position de rotation, une image du produit en verre est capturée, dans lequel, sur l'image, une marque (M) prédéterminée sur le produit en verre, telle qu'une ligne et/ou un point, est détectée afin de déterminer la position de rotation.

13. Procédé selon l'une quelconque des revendications précédentes, **caractérisé en ce que** l'inspection comprend en outre un contrôle moyennant quoi au moins une étape des étapes a., b. et c. est ajustée sur la base des résultats de mesure obtenus avec l'aide de l'au moins un capteur et, possiblement, de la position de rotation déterminée, plus particulièrement sur la base de la répartition d'épaisseur de verre latérale déterminée du produit virtuel.

14. Procédé de production et d'inspection de produits en verre creux (4) en matériau de produit en verre, dans lequel les produits en verre sont au moins fabriqués en :
a. chauffant le matériau de produit en verre ;
b. formant le matériau de produit en verre chauffé en au moins un produit en verre dans un flux de production ;
c. refroidissant le produit en verre formé ; **caractérisé en ce que** l'inspection des produits en verre est effectuée selon l'une quelconque des revendications 1 à 13.

15. Système (1) d'inspection de produits en verre (4) en matériau de produit en verre selon le procédé selon la partie de pré-caractérisation de la revendication 1, dans lequel le système comprend :
au moins un capteur (10) destiné à scanner les produits en verre ; et
une unité de traitement de signal (14) reliée à l'au moins un capteur afin de traiter les signaux qui proviennent de l'au moins un capteur, **caractérisé en ce que** l'au moins un capteur est un capteur, tel qu'un capteur confocal chromatique, destiné à mesurer une épaisseur du verre, dans lequel le système comprend en outre une unité de position de rotation (11) telle qu'une caméra destinée à obtenir des informations qui représentent une position de rotation (R) par rapport à l'au moins un capteur et autour d'un axe axial (A) d'un produit en verre sur lequel les mesures ont été effectuées avec l'au moins un capteur, dans lequel, pendant l'utilisation, l'au moins un capteur est configuré de sorte que les produits en verre soient successivement mesurés avec l'au moins un capteur, dans lequel l'unité de traitement de signal est configurée pour, pour chaque produit scanné, sur la base des résultats de mesure de l'au moins un capteur et des informations associées de l'unité de position de rotation, déterminer une répartition d'épaisseur de verre latérale autour d'un axe axial d'un produit en verre virtuel (4') qui représente la pluralité de produits en verre de sorte que le système est en outre configuré pour exécuter le procédé selon la partie de caractérisation de la revendication 1.

16. Système (1) de production et d'inspection de produits en verre (4) en matériau de produit en verre selon le procédé de la partie de pré-caractérisation de la revendication 14, comprenant le système selon la revendication 15, dans lequel le système comprend en outre :
un appareil de chauffage (2) destiné à exécuter l'étape a. ;
un appareil de formation de produits (3) tel qu'un moule (104) destiné à exécuter l'étape b. ;
un appareil de refroidissement (7) destiné à exécuter l'étape c. ; et
un appareil de transport (6) destiné à transporter les produits en verre formés avec l'appareil de formation de produits depuis l'appareil de formation de produits vers l'appareil de refroidissement.

17. Système selon la revendication 15 ou 16, **caractérisé en ce que** le système est en outre configuré pour exécuter le procédé selon l'une quelconque des revendications 2 à 13.

18. Système selon l'une des revendications précédentes 15, 16 ou 17, **caractérisé en ce que** le système est configuré de sorte que, pendant l'utilisation, plusieurs des étapes b. soient exécutées parallèlement les unes aux autres afin de produire, parallèlement les unes aux autres, une pluralité de produits en verre dans une pluralité de flux de production (107) qui comprennent chacun une étape b., dans lequel l'unité de traitement de signal (14) est configurée de sorte que, pendant l'utilisation, les épaisseurs de verre déterminées et les positions de rotation associées de la pluralité de produits en verre qui proviennent d'un même flux de production soient traitées en combinaison afin d'obtenir des informations sur une répartition d'épaisseur de verre latérale autour d'un axe axial d'un produit en verre virtuel (4') qui représente la pluralité de produits en verre issus d'un même flux de production.
